(19) Europäisches Patentamt
European Patent Office
Office européen des brevets

(11) EP 2 079 437 B1

# (12) EUROPEAN PATENT SPECIFICATION

(45) Date of publication and mention of the grant of the patent:
**17.08.2016 Bulletin 2016/33**

(21) Application number: **07802860.2**

(22) Date of filing: **24.08.2007**

(51) Int Cl.:
*A61K 8/73* (2006.01) *A61K 8/81* (2006.01)
*A61K 8/85* (2006.01) *A61K 8/89* (2006.01)
*A61K 8/92* (2006.01) *A61Q 1/10* (2006.01)
*A61K 8/31* (2006.01) *A61K 8/90* (2006.01)
*A61K 8/02* (2006.01) *A61K 8/18* (2006.01)
*A61K 8/891* (2006.01) *A61K 8/893* (2006.01)

(86) International application number:
**PCT/EP2007/058811**

(87) International publication number:
**WO 2008/023056 (28.02.2008 Gazette 2008/09)**

## (54) PROCESS FOR MAKING UP KERATIN FIBRES AND PACKAGING ASSEMBLY

VERFAHREN ZUR KOSMETISCHEN BEHANDLUNG VON KERATINFASERN UND VERPACKUNGSANORDNUNG

PROCÉDÉ DE MAQUILLAGE DE FIBRES DE KÉRATINE ET ENSEMBLE DE CONDITIONNEMENT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **25.08.2006 FR 0653468**
**01.09.2006 US 841553 P**

(43) Date of publication of application:
**22.07.2009 Bulletin 2009/30**

(73) Proprietor: **L'Oréal**
**75008 Paris (FR)**

(72) Inventors:
- **RAINEAU, Olivier**
  **F-75013 Paris (FR)**
- **PAYS, Karl**
  **F-94410 Saint Maurice (FR)**
- **BEZACIER-MAHIET, Anais**
  **F-26400 Crest (FR)**
- **ARDITTY, Stéphane**
  **F-91160 Ballainvilliers (FR)**

(74) Representative: **Goudet, Sylvain**
**L'Oréal**
**Service DIPI**
**9 Rue Pierre Dreyfus**
**92110 Clichy (FR)**

(56) References cited:
**EP-A1- 1 430 868** **FR-A- 2 864 894**
**US-A- 3 384 547** **US-A- 5 389 363**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

The present invention relates to a cosmetic process for making up or for the non-therapeutic care of keratin fibres (such as the eyelashes, the eyebrows or the hair), comprising the application to the keratin fibres of a cosmetic composition having a threading nature dmax of greater than or equal to 5 mm.

It is in particular in the form of a mascara, or of an eyebrow product. More especially, the invention relates to a mascara.

The term "mascara" is intended to mean a composition intended to be applied to keratin fibres: it may be a makeup composition for keratin fibres, a makeup base for keratin fibres or base-coat, a composition to be applied to a mascara, also referred to as top-coat, or else a composition for the cosmetic treatment of keratin fibres. The mascara is more particularly intended for keratin fibres of human beings, but also for false eyelashes.

In order to obtain an eyelash-lengthening effect, mascara compositions comprising fibres are known from the prior art. These fibres can add a small amount of physical length to the eyelashes when they are sufficiently rigid and visible and they are at the end of the eyelash. However, the gain in physical lengthening obtained via such mascaras remains moderate since it is difficult to orient the fibres so as to stack them at the end of the eyelash. Furthermore, the presence of fibres can reduce the adhesion of the mascara to the eyelashes, lengthening the time necessary for applying the makeup.

Another technical approach, described in document EP 1430868, is the use of mascaras having a "threading" nature at ambient temperature, and which are capable of forming, when applied to keratin fibres and once they have been drawn with a brush, threads in the elongation of the eyelashes, without the use of a source of heat.

However, the ability of these mascaras to thread at ambient temperature does not simplify their use: in particular, when it is sampled in order to be applied, the mascara can form threads between the container that contains it and the applicator or between the eyelashes and the applicator.

Furthermore, it is tricky to control the length of the threads formed on the eyelashes since the threads do not break spontaneously. In addition, said threads are rarely of sufficient rigidity to remain aligned in the elongation of the eyelash and to allow a long-lasting lengthening effect.

The aim of the present invention is to provide a process for coating keratin fibres which allows a good eyelash-lengthening effect, in particular under the action of heat, and with good hold over time.

The inventors have discovered that the properties described above can be obtained using a composition having a specific threading nature under the action of a source of heat.

More specifically, a subject of the invention is a cosmetic process for making up or for the non-therapeutic care of keratin fibres, comprising the application to the keratin fibres of a cosmetic composition according to claim 1.

The threading nature represents the ability of the composition, once subjected to a source of heat, to form, on the keratin fibres, threads which, once they have been drawn with an applicator, are sufficiently consistent and conserve their shape. The use of heat so as to bring the composition to a temperature greater than or equal to 40°C makes it possible to control the length of the threads formed in the extension of the eyelashes.

In particular, after application of the softened composition and drawing of the threads, the latter solidify at ambient temperature along the extension of each eyelash and make it possible to obtain a notable lengthening effect.

The composition can in particular be used in combination with a heating instrument, in particular a heating brush, which can be applied to the eyelashes before, during or after the latter are coated with the composition, or conditioned in a device for applying the composition while hot.

According to one embodiment, the composition as defined above is applied to the upper end of the keratin fibres, in particular the eyelashes.

A subject of the present invention is also a packaging and application assembly for a composition for making up and/or for the care of keratin fibres, in particular the eyelashes or the eyebrows, according to claim 22.

The composition can be brought to a temperature of greater than or equal to 40°C prior to, simultaneously with or subsequent to its application, in particular by means of an application device comprising heating means, such as a heating brush.

## Measurement of the threading nature

The threading nature of the composition is determined using the texturometer sold under the name TA X- T2i by the company RHEO, equipped with a temperature-controlled spindle, this spindle being a stainless-steel heating cartridge of reference Firerod DIV-STL (company Watlow, France), 3.17 mm in diameter and 60 mm in length, having a maximum power of 40W at a voltage of 24V, with a type K loc C thermocouple.

The heating cartridge is powered by a 5V/0.5A LKS 005-5V direct current source from Elka-Electronique. Its temperature is regulated by a PID TC48 controller from Faucigny Instrument (France). An attachment lengthening piece was created in order to attach the temperature-controlled spindle to the measuring arm of the texturometer.

The measurement is carried out on threads of composition obtained by imposing a vertical displacement of the spindle until contact with a sample of the composition and then, after a waiting period in contact, by imposing a vertical displacement of the spindle upwards. Since the composition has a threading nature when hot, a thread forms between the spindle in the withdrawal phase and the sample of the composition, said thread becoming more consistent under the effect of the cooling in ambient air. The measurement of dmax consists in measuring the length of the threads thus formed after detachment from the surface of the spindle.

The protocol is as follows:

a) a sample of the composition is prepared by filling, to its maximum, a stainless-steel cupule 2 mm thick and 20 mm in diameter, the excess composition being levelled at the surface,
b) the temperature of the spindle is controlled at 40°C,
c) the spindle descends at a speed of 10 mm/s until contact with the surface of the composition,
d) the spindle is kept fixed for 10 s and then is raised again at a speed of 10 mm/s.

During the spindle withdrawal phase, a thread is formed between the composition and the spindle. As the spindle moves away from the surface of the composition, the thread formed cools and becomes more consistent. Starting from a certain elongation, the thread detaches from the spindle.

The threading nature or dmax (expressed in mm) corresponds to the length of the thread obtained after breaking, measured with a graduated rule.

The measurement of the threading nature is repeated three times for the same composition, at different sites of the cupule, and a mean "threading" dmax is calculated for each composition.

Steps b) to d) are repeated for the same composition at a fixed spindle temperature in step b) respectively of 50°C, 60°C, 70°C, 80°C, 90°C, 100°C, 110°C, 120°C, 130°C and 140°C.

Among the threading values that may be obtained at the various temperatures, the highest value is retained as threading nature value dmax.

The composition used in the process according to the invention has a threading nature dmax of greater than or equal to 5 mm, which can range up to 100 mm, preferably greater than or equal to 7 mm, better still greater than or equal to 10 mm, and better still greater than or equal to 15 mm.

Preferably, the composition is capable of forming a thread such that, if, after formation of the thread and measurement of the dmax according to the protocol indicated above, the cupule comprising the composition is placed vertically (so that the thread is in the horizontal position, i.e. subjected to gravity) for at least 30 seconds, the thread conserves a minimum length of 5 mm (measurable manually with a graduated rule).

The composition having a threading nature according to the invention makes it possible to obtain, during the application to keratin fibres, a thread of composition along the extension of the eyelash. This thread conserves its shape, remains rigid and does not shrink, thereby making it possible to obtain an eyelash-lengthening effect.

The composition used in the process according to the invention is heated to a temperature of greater than or equal to 40°C, preferably greater than or equal to 45°C, better still greater than or equal to 50°C, and even better still greater than or equal to 60°C.

The temperature can range up to 150°C, preferably up to 120°C, better still up to 100°C, even better still up to 95°C.

Preferably, the composition is brought to the temperature at which it has the threading nature dmax, measured as indicated above (i.e. to the temperature at which the threading nature is highest).

In particular, the compound itself has a threading nature dmax, as defined above, of greater than or equal to 5 mm, which can range up to 100 mm, preferably greater than or equal to 7 mm, better still greater than or equal to 10 mm, and better still greater than or equal to 15 mm, when it is brought to a temperature of greater than or equal to 40°C.

For this reason, a subject of the invention is also a cosmetic process for making up or for the non-therapeutic care of keratin fibres, comprising the application to the keratin fibres of a cosmetic composition containing a compound or a mixture of compounds, said compound or mixture of compounds having a threading nature dmax of greater than or equal to 5 mm, when it is brought to a temperature of greater than or equal to 40°C, and said composition being, prior to, simultaneously with or subsequent to its application, brought to a temperature of greater than or equal to 40°C.

The composition comprises a physiologically acceptable medium, i.e. a medium which is non-toxic and can be applied to keratin fibres, such as the eyelashes, the eyebrows and the hair of human beings, in particular compatible with the ocular region.

According to one embodiment, the process according to the invention consists in:

- applying, to the keratin fibres, a coloured cosmetic composition (i.e. comprising at least one dyestuff as defined below) containing at least one compound or a mixture of compounds, which, when the composition is brought to a temperature of greater than or equal to 40°C, confers on said composition a threading nature dmax of greater than

or equal to 5 mm, then

- in bringing said composition, prior to, simultaneously with or subsequent to its application, to a temperature of greater than or equal to 40°C so as to form coloured threads at the end of the eyelashes.

According to another embodiment, the process according to the invention consists in:

- applying a non-coloured cosmetic composition to the keratin fibres, said composition containing at least one compound or a mixture of compounds which, when the composition is brought to a temperature of greater than or equal to 40°C, confers on said composition a threading nature dmax of greater than or equal to 5 mm,
- bringing said composition, prior to, simultaneously with or subsequent to its application, to a temperature of greater than or equal to 40°C so as to form colourless threads at the end of the eyelashes,
- and then, after cooling of the threads, making up the keratin fibres and the colourless threads set in their extension, with a second composition comprising one or more dyestuffs, so as to colour the threads.

In particular, the composition containing at least one compound or a mixture of compounds which, when the composition is brought to a temperature of greater than or equal to 40°C, confers on said composition a threading nature dmax of greater than or equal to 5 mm, is applied to the upper end of the eyelashes.

It is thus possible, according to this embodiment, to make up the colourless threads formed on the eyelashes or at the end of the eyelashes (eyelash extension) with a deposit of a second coloured composition, and then to remove said deposit of second composition alone, without removing the makeup from the assembly of the eyelashes and the extensions, so as to be able to subsequently again make up the threads formed at the end of the eyelashes, for example with another composition of a different colour.

**Composition capable of conferring on said composition a threading nature dmax of greater than or equal to 5 mm**

The composition advantageously comprises at least one compound which confers on said composition a threading nature dmax of greater than or equal to 5 mm, or a mixture of compounds such that said mixture confers on said composition a threading nature dmax of greater than or equal to 5 mm, when said composition is heated to a temperature of greater than or equal to 40°C.

This compound may be hydrocarbon-based or silicone-based and advantageously has a thermoplastic behaviour.

This compound is preferably solid at ambient temperature. Advantageously, it itself has a threading nature dmax, measured according to the protocol mentioned above, of greater than or equal to 5 mm when it is brought to a temperature of greater than or equal to 40°C, i.e. it is capable of producing threads as described above, at a temperature of greater than or equal to 40°C, for example ranging from 40 to 150°C, preferably greater than or equal to 45°C, for example ranging from 45 to 120°C, better still greater than or equal to 50°C, for example ranging from 50 to 100°C, and even better still greater than or equal to 60°C.

The compound can have a threading nature dmax of greater than or equal to 5 mm, which can range up to 100 mm, preferably greater than or equal to 7 mm, better still greater than or equal to 10 mm, and better still greater than or equal to 15 mm.

Preferably, the compound is itself capable of forming a thread such that, if, after formation of the thread and measurement of the dmax according to the protocol indicated above, the cupule comprising the compound is placed vertically (such that the thread is in the horizontal position, i.e. subjected to gravity) for at least 30 seconds, the thread conserves a minimum length of 5 mm (which can be measured manually with a graduated rule).

This compound is preferably a polymer and can be advantageously chosen from:

A/ Polymers and copolymers comprising at least one alkene monomer, in particular ethylene-based copolymers. Such compounds can be chosen from:

- copolymers of an alkene and of vinyl acetate, in particular copolymers of ethylene and of vinyl acetate.
  Copolymers of ethylene and of vinyl acetate preferably comprising more than 25% by weight, for example from 25 to 50% by weight, preferably from 25 to 35% by weight, for example about 28% by weight, of vinyl acetate relative to the total weight of the polymer are in particular used.
  As an example of ethylene/vinyl acetate copolymers, mention may be made of those which are sold under the name Elvax by the company DuPont de Nemours, and in particular the compounds Elvax 40W, Elvax 140W, Elvax 200W, Elvax 205W, Elvax 210W and Elvax 310.
  Mention may also be made of the products sold under the name Evatane by the company Arkema, such as Evatane 28-800. Mention may also be made of Melthene-H Grade H-6410M offered by the company Tosoh

Polymer;
These copolymers can present an weight-average mass (Mw) ranging from 50000 to 80000, better, from 60 000 to 70 000, and even better from 63000 to 73000

- copolymers of ethylene and of octene, such as, for example, the products sold under the reference "Affinity" by the company Dow Plastics, for instance Affinity GA 1900 GA 1950.
These polymers and copolymers can be used alone or as a mixture with at least one compound chosen from "tackifying" resins as described in the Handbook of Pressure Sensitive Adhesives, edited by Donatas Satas, 3rd edition, 1989, p. 609-619, the waxes as described below, and combinations thereof. The tackifying resins can in particular be chosen from rosin, rosin derivatives, hydrocarbon-based resins, and mixtures thereof. Mention may in particular be made of indene hydrocarbon-based resins such as the resins derived from the polymerization of a predominant proportion of indene monomer and of a minor proportion of monomers chosen from styrene, methylindene, methylstyrene and mixtures thereof. These resins can optionally be hydrogenated. They can have a molecular weight ranging from 290 to 1150. As examples of indene resins, mention may in particular be made of the hydrogenated indene/methylstyrene/styrene copolymers sold under the name "Regalite" by the company Eastman Chemical, in particular Regalite R 1100, Regalite R 1090, Regalite R-7100, Regalite R1010 Hydrocarbon Resin and Regalite R1125 Hydrocarbon Resin.
As a mixture based on ethylene/vinyl acetate copolymer, mention may, for example, be made of the products sold under the name Coolbind by the company National Starch.
These polymers can be in pure form or can be carried in an aqueous phase or an organic solvent phase.

B/ Polyvinyl acetate homopolymers preferably having a molecular weight of less than 20 000, for instance Raviflex BL1S from the company Vinavil.

C/ Silicone T resins

These resins are crosslinked organosiloxane polymers.

Silicone resin nomenclature is known as "MDTQ", the resin being described according to the various siloxane monomeric units that it comprises, each of the letters "MDTQ" characterizing a type of unit.

The letter M represents the monofunctional unit of formula $(CH_3)_3SiO_{1/2}$, the silicon atom being attached to a single oxygen atom in the polymer comprising this unit.

The letter D signifies a difunctional unit $(CH_3)_2SiO_{2/2}$ in which the silicon atom is attached to two oxygen atoms.

The letter T represents a trifunctional unit of formula $(CH_3)SiO_{3/2.}$

In the units M, D and T defined above, at least one of the methyl groups can be substituted with a group R different from the methyl group, such as a hydrocarbon-based (in particular alkyl) radical containing from 2 to 10 carbon atoms or a phenyl group or else a hydroxyl group.

Finally, the letter Q signifies a tetrafunctional unit $SiO_{4/2}$ in which the silicon atom is attached to four hydrogen atoms, themselves attached to the rest of the polymer.

Mention is made of T resins, in particular functionalized T silicone resins such as polyphenylsiloxanes, in particular functionalized with silanol (Si-OH) groups, such as that sold under the reference Dow Corning (R)Z-1806.

D/ Film-forming ethylenic block polymers

These polymers comprise at least a first block and at least a second block having different glass transition temperatures (Tg), said first and second blocks being connected to one another by means of an intermediate block comprising at least one monomer constituting the first block and at least one monomer constituting the second block.

Advantageously, the first and second blocks of the block polymer are compatible with one another.

Such polymers are described, for example, in documents EP 1411069, WO 04/028488 or WO 04/028493.

The term "block" polymer is intended to mean a polymer comprising at least 2 distinct blocks, for example at least 3 distinct blocks.

The first and second blocks of the polymer differ from one another by virtue of their degree of deformability. Thus, the first block may be rigid and the second block may be flexible.

The glass transition temperatures of the flexible and rigid blocks may be theoretical Tgs as determined from the theoretical Tgs of the monomers constituting each of the blocks, which can be found in a reference manual such as the Polymer Handbook, 3rd ed., 1989, John Wiley, according to the following relationship, known as Fox's law:

$$1/Tg = \sum_i (\omega_i/Tg_i),$$

$\omega_i$ being the mass fraction of the monomer i in the block under consideration and $Tg_i$ being the glass transition temperature of the homopolymer of the monomer i.

Unless otherwise indicated, the Tgs indicated in the first and second blocks in the present application are theoretical Tgs.

The rigid block can have a Tg of greater than 20°C.

The flexible block can have a Tg of less than or equal to 20°C.

According to one embodiment, the copolymer comprises a first rigid block and a second flexible block.

Preferably, the proportion of the rigid block ranges from 20% to 90% by weight of the copolymer, better still from 30% to 90%, and even better still from 50% to 90%.

Preferably, the proportion of the flexible block ranges from 5% to 75% by weight of the copolymer, preferably from 10% to 50%, and better still from 15% to 45%.

Rigid block

In the context of the present invention, the rigid block(s) is (are) formed from the following monomers:

- methacrylates of formula $CH_2 = C(CH_3)\text{-}COOR_1$ in which $R_1$ represents an isobornyl group,
- acrylates of formula $CH_2 = CH\text{-}COOR_2$ in which $R_2$ represents an isobornyl group,

Monomers of the rigid block are isobornyl methacrylate, isobornyl acrylate, and mixtures thereof.

Flexible block

In the context of the present invention, the flexible block(s) is (are) formed from the following monomers:

isobutyl acrylate.

Each of the sequences can contain a minority proportion of at least one monomer constituting the other block. Thus, the first block can contain at least one monomer constituting the second block, and conversely.
Each of the first and/or second block can comprise, in addition to the monomers indicated above, one or more other monomers, called additional monomers, different from the main monomers mentioned above.
This additional monomer is, for example, chosen from:

a) hydrophilic monomers such as:

- monomers comprising one or more ethylenic unsaturation(s), other than acrylic acid, comprising at least one carboxylic or sulphonic acid function, for instance methacrylic acid, crotonic acid, maleic anhydride, itaconic acid, fumaric acid, maleic acid, acrylamidopropane-sulphonic acid, vinylbenzoic acid, vinyl-phosphoric acid, and the salts thereof,
- monomers comprising one or more ethylenic unsaturation(s) and comprising at least one tertiary amine

function such as 2-vinylpyridine, 4-vinylpyridine, dimethylaminoethyl methacrylate, diethylaminoethyl methacrylate, dimethylaminopropylmethacrylamide, and the salts thereof,

- methacrylates of formula $CH_2 = C(CH_3)-COOR_6$ in which $R_6$ represents a linear or branched alkyl group containing from 1 to 4 carbon atoms, such as a methyl, ethyl, propyl or isobutyl group, said alkyl group being substituted with one or more substituents chosen from hydroxyl groups (such as 2-hydroxy-propyl methacrylate or 2-hydroxyethyl methacrylate) and halogen atoms (Cl. Br, I, F), such as trifluoroethyl methacrylate,
- methacrylates of formula $CH_2 = C(CH_3)-COOR_9$, $R_9$ representing a linear or branched $c_6$ to $C_{12}$ alkyl group in which one or more heteroatoms chosen from O N and S is (are) optionally intercalated, said alkyl group being substituted with one or more substituents chosen from hydroxyl groups and halogen atoms (Cl, Br, I, F);
- acrylates of formula $CH_2 = CHCOOR_{10}$, $R_{10}$ representing a linear or branched $C_1$ to $C_{12}$ alkyl group substituted with one or more substituents chosen from hydroxyl groups and halogen atoms (Cl, Br, I and F), such as 2-hydroxypropyl acrylate and 2-hydroxyethyl acrylate, or $R_{10}$ represents a ($C_1$-$C_{12}$) alkyl-O-POE (polyoxyethylene) with repetition of the oxyethylene unit from 5 to 30 times, for example methoxy-POE, or $R_{10}$ represents a polyoxyethylenated group comprising from 5 to 30 ethylene oxide units,

b) monomers comprising one or more ethylenic unsaturation(s) and comprising one or more silicon atoms, such as methacryloxypropyltrimethoxysilane or methacryloxypropyltris-(trimethylsiloxy)silane,

- and mixture thereof.

This or these additional monomer(s) generally represent (s) an amount of less than or equal to 30% by weight, for example from 1% to 30% by weight, preferably from 5% to 20% by weight, and even more preferably from 7% to 15% by weight, of the total weight of the first and/or second blocks.
According to one embodiment, the copolymer can comprise at least a first block and at least a second block connected to one another by means of an intermediate segment comprising at least one monomer constituting the first block and at least one monomer constituting the second block.
Preferably, the intermediate block is essentially derived from monomers constituting the first block and the second block.
Advantageously, the intermediate segment comprising at least one monomer constituting the first block and at least one monomer constituting the second block of the copolymer is a random polymer.
The copolymer is essentially derived from monomers chosen from alkyl methacrylates, alkyl acrylates, and mixtures thereof.
In the above and subsequent text, the term "essentially" is intended to mean comprising at least 85%, preferably at least 90%, better still at least 95%, and even better still 100%.
As regards the acrylate and methacrylate esters they derive from the esterification of isoborneol.
According to the invention, said copolymer comprises at least acrylate and methacrylate monomers deriving from the esterification of isoborneol.
According to the invention, the film-forming linear block polymer comprises at least isobornyl acrylate monomers, at least isobornyl methacrylate monomers and at least isobutyl acrylate monomers.
The block polymer comprises at least:

- a rigid block, which is an isobornyl methacrylate/isobornyl acrylate copolymer, and
- a flexible block, which is an isobutyl acrylate copolymer.

More specifically, the copolymer can comprise 50% to 80% by weight of isobornyl methacrylate/- acrylate and from 10% to 20% by weight of isobutyl acrylate.
The weight-average mass (Mw) of the copolymer preferably ranges from 80 000 to 300 000, or even from 100 000 to 150 000.
The number-average mass (Mn) of the copolymer preferably ranges from 20 000 to 90 000; it ranges, for example, from 25 000 to 45 000.

E/ Copolymers of butadiene and of styrene.
Mention may in particular be made of the styrene/- butadiene copolymers sold under the reference Pliolite S5E by the company Eliokem.

F/ Polyesters comprising at least one monomer bearing at least one $-SO_3M$ group (M representing a hydrogen atom, an $NH_4^+$ ammonium ion or a metal ion), also called sulphopolyesters.

These polyesters advantageously have a glass transition temperature (Tg) of greater than 38°C. They can have a weight-average molecular mass of advantageously less than 200 000, for example ranging from 10 000 to 50 000.
These polyesters are obtained, in a known manner, by polycondensation of at least one dicarboxylic acid with at least one polyol, in particular diols.
The dicarboxylic acid is made of isophthalic acid.
The diol is made of cyclohexanedimethanol.
The polyester comprises at least one monomer bearing at least one $-SO_3M$ group, with M representing a hydrogen atom, an $NH_4^+$ ammonium ion or a metal ion, for instance an $Na^+$, $Li^+$, $K^+$, $Mg^{2+}$, $Ca^{2+}$, $Cu^{2+}$, $Fe^{2+}$ or $Fe^{3+}$ ion. A bifunctional aromatic monomer comprising such an $-SO_3M$ group is used.
The aromatic ring of the bifunctional aromatic monomer also bearing an $-SO_3M$ group as described above is made of sulphoisophthalic acid.
Use is made of copolymers based on isophthalate/sulphoisophthalate, and according tp the invention of copolymers obtained by condensation of diethylene glycol, cyclohexane dimethanol, isophthalic acid and sulphoisophthalic acid. Such polymers are sold, for example, under the trade mark Eastman AQ® by the company NOVEON, for instance Eastman AQ 38S.

According to a specific embodiment, the mixture of compounds which confers on the composition a threading nature dmax of greater than or equal to 5 mm can be a mixture of wax, especially paraffin wax, and ethylene/vinyle acetate copolymer.
Said mixture can comprise from 50 to 65% by weight ethylene/vinyle acetate copolymer related to the total weight of the mixture and from 35 to 50% by weight of wax, related to the total weight of the mixture.
Preferably, the ethylene/vinyle acetate copolymer comprises more than 25% by weight of vinyl acetate relative to the total weight of the polymer, for example about 28% by weight of vinyl acetate.
Preferably, the ethylene/vinyle acetate copolymer has a weight-average mass (Mw) ranging from 50000 to 80000, better, from 60 000 to 70 000, and even better from 63000 to 73000.
The compound or the mixture of compounds which confers on the composition a threading nature dmax of greater than or equal to 5 mm can be present in the composition at a solids content of at least 5% by weight relative to the total weight of the composition, for example ranging from 5% to 100% by weight relative to the total weight of the composition, preferably ranging from 10% to 100% by weight, and better still from 12% to 100% by weight.
According to one embodiment, the compound or the mixture of compounds conferring on the composition a threading nature dmax of greater than or equal to 5 mm is present at a content of greater than or equal to 20% by weight relative to the total weight of the composition, preferably greater than or equal to 25% by weight, better still greater than or equal to 30% by weight, and even better still greater than or equal to 40%, or even greater than or equal to 50% by weight.

## Aqueous phase

The composition according to the invention can preferably comprise an aqueous medium, constituting an aqueous phase, which can form the continuous phase of the composition.
The aqueous phase of the composition according to the invention is advantageously a continuous aqueous phase.
The expression "composition comprising a continuous aqueous phase" is intended to mean that the composition has a conductivity, measured at 25°C, of greater than 23 μS/cm (microSiemens/cm), the conductivity being measured, for example, using an MPC227 conductivity meter from Mettler Toledo and an Inlab730 conductivity measuring cell. The measuring cell is immersed in the composition, so as to eliminate the air bubbles that may form between the 2 electrodes of the cell. The conductivity is read as soon as the conductivity meter value has become stabilized. An average is produced over at least 3 successive measurements.
The aqueous phase can consist essentially of water; it can also comprise a mixture of water and of water-miscible solvent (water-miscibility greater than 50% by weight at 25°C), for instance lower monoalcohols having from 1 to 5 carbon atoms, such as ethanol or isopropanol, glycols having from 2 to 8 carbon atoms, such as propylene glycol, ethylene glycol, 1,3-butylene glycol or dipropylene glycol, $C_3$-$C_4$ ketones, $C_2$-$C_4$ aldehydes, and mixtures thereof.
The aqueous phase (water and, optionally, water-miscible solvent) can be present at a content ranging from 1% to 95% by weight, relative to the total weight of the composition, preferably ranging from 3% to 80% by weight, and preferably ranging from 5% to 60% by weight.

### Emulsifying system

The compositions according to the invention can contain emulsifying surfactants present in particular in a proportion ranging from 0.1% to 20%, and better still from 0.3% to 15% by weight relative to the total weight of the

composition.

According to the invention, use is generally made of an emulsifier appropriately chosen in order to obtain an oil-in-water emulsion. In particular, use may be made of an emulsifier having, at 25°C, an HLB (hydrophilic-lipophilic balance) balance within the meaning of Griffin, of greater than or equal to 8.

The HLB value according to Griffin is defined in J. Soc. Cosm. Chem. 1954 (Volume 5), pages 249-256.

These surfactants can be chosen from non-ionic, anionic, cationic or amphoteric surfactants or also surfactant emulsifiers. Reference may be made to the document "Encyclopedia of Chemical Technology, Kirk-Othmer" Volume 22, p. 333-432, 3rd edition, 1979, Wiley, for the definition of the properties and functions (emulsifying) of surfactants, in particular p. 347-377 of this reference, for anionic, amphoteric and non-ionic surfactants.

The surfactants preferably used in the composition according to the invention are chosen from:

a) non-ionic surfactants with an HLB of greater than or equal to 8 to 25°C, used alone or as a mixture; mention may in particular be made of:

- oxyethylenated and/or oxypropylenated ethers (which can comprise from 1 to 150 oxyethylenated and/or oxypropylenated groups) of glycerol;
- oxyethylenated and/or oxypropylenated ethers (which can comprise from 1 to 150 oxyethylenated and/or oxypropylenated groups) of fatty alcohols (in particular of $C_8$-$C_{24}$ and preferably $C_{12}$-$C_{18}$ alcohols), such as the oxyethylenated ether of stearyl alcohol comprising 20 oxyethylenated groups (CTFA name "Steareth-20"), such as Brij 78 sold by the company Uniquema, the oxyethylenated ether of cetearyl alcohol comprising 30 oxyethylenated groups (CTFA name "Ceteareth-30") and the oxyethylenated ether of the mixture of $C_{12}$-$C_{15}$ fatty alcohols comprising 7 oxyethylenated groups (CTFA name "C12-15 Pareth-7") sold under the name Neodol 25-7® by Shell Chemicals,
- esters of a fatty acid (in particular of a $C_8$-$C_{24}$ and preferably $C_{16}$-$C_{22}$ acid) and of polyethylene glycol (which can comprise from 1 to 150 ethylene glycol units), such as PEG-50 stearate and PEG-40 monostearate, sold under the name Myrj 52P® by the company ICI Uniquema,
- esters of a fatty acid (in particular of a $C_8$-$C_{24}$ and preferably $C_{16}$-$C_{22}$ acid) and of oxyethylenated and/or oxypropylenated glycerol ethers (which can comprise from 1 to 150 oxyethylenated and/or oxypropylenated groups), such as the PEG-200 glyceryl monostearate sold under the name Simulsol 220 TM® by the company Seppic; polyethoxylated glyceryl stearate comprising 30 ethylene oxide groups, such as the product Tagat S® sold by the company Goldschmidt, polyethoxylated glyceryl oleate comprising 30 ethylene oxide groups, such as the product Tagat O® sold by the company Goldschmidt, polyethoxylated glyceryl cocoate comprising 30 ethylene oxide groups, such as the product Varionic LI 13® sold by the company Sherex, polyethoxylated glyceryl isostearate comprising 30 ethylene oxide groups, such as the product Tagat L® sold by the company Goldschmidt, and polyethoxylated glyceryl laurate comprising 30 ethylene oxide groups, such as the product Tagat I® from the company Goldschmidt,
- esters of a fatty acid (in particular of a $C_8$-$C_{24}$ and preferably $C_{16}$-$C_{22}$ acid) and of oxyethylenated and/or oxypropylenated sorbitol ethers (which can comprise from 1 to 150 oxyethylenated and/or oxypropylenated groups), such as the polysorbate 60 sold under the name Tween 60® by the company Uniquema,
- dimethicone copolyol, such as that sold under the name Q2-5220® by the company Dow Corning,
- dimethicone copolyol benzoate (FINSOLV SLB 101® and 201® from the company Fintex),
- copolymers of propylene oxide and of ethylene oxide, also known as EO/PO polycondensates,
- and mixtures thereof.

The EO/PO polycondensates are more particularly copolymers consisting of polyethylene glycol and polypropylene glycol blocks, for instance polyethylene glycol/polypropylene glycol/polyethylene glycol triblock polycondensates. These triblock polycondensates have, for example, the following chemical structure:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

in which formula a ranges from 2 to 120, and b ranges from 1 to 100.

The EO/PO polycondensate preferably has a weight-average molecular weight ranging from 1000 to 15 000, and better still ranging from 2000 to 13 000. Advantageously, said EO/PO polycondensate has a cloud point, at 10 g/1 in distilled water, of greater than or equal to 20°C, preferably greater than or equal to 60°C. The cloud point is measured according to standard ISO 1065. As EO/PO polycondensate that can be used according to the invention, mention may be made of the polyethylene glycol/polypropylene glycol/polyethylene glycol triblock polycondensates sold under the names Synperonic®, such as Synperonic PE/L44® and Synperonic PE/F127®, by the company ICI;

b) non-ionic surfactants with an HLB of less than 8 at 25°C, optionally in combination with one or more non-ionic surfactants with an HLB of greater than 8 at 25°C, as mentioned above, such as:

esters and ethers of monosaccharides, such as sucrose stearate, sucrose cocoate, sorbitan stearate and mixtures thereof, such as Arlatone 2121® sold by the company ICI or SPAN 65V from the company Uniquema;
esters of fatty acids (in particular of a $C_8$-$C_{24}$ and preferably $C_{16}$-$C_{22}$ acid) and of a polyol, in particular of glycerol or of sorbitol, such as glyceryl stearate, for instance the product sold under the name Tegin M® by the company Goldschmidt, glyceryl laurate, for instance the product sold under the name Imwitor 312® by the company Hüls, polyglyceryl-2 stearate, sorbitan tristearate or glyceryl ricinoleate;
oxyethylenated and/or oxypropylenated ethers, such as the oxyethylenated ether of stearyl alcohol comprising 2 oxyethylenated groups (CTFA name "Steareth-2"), for instance Brij 72 sold by the company Uniquema;
the cyclomethicone/dimethicone copolyol mixture sold under the name Q2-3225C® by the company Dow Corning;

c) anionic surfactants such as:

salts of polyoxyethylenated fatty acids, in particular those derived from amines or the alkali metal salts and mixtures thereof;
phosphoric esters and their salts, such as "DEA oleth-10 phosphate" (Crodafos N 10N from the company Croda) or monopotassium monocetyl phosphate (Amphisol K from Givaudan or Arlatone MAP 160K by the company Uniquema);
sulphosuccinates, such as "Disodium PEG-5 citrate lauryl sulphosuccinate" and "Disodium ricinoleamido MEA sulphosuccinate";
alkyl ether sulphates, such as sodium lauryl ether sulphate;
isethionates;
acylglutamates, such as "Disodium hydrogenated tallow glutamate" (Amisoft HS-21 R® sold by the company Ajinomoto), and mixtures thereof.

By way of representation of cationic surfactants, mention may in particular be made of:

- alkyl imidazolidiniums, such as isostearyl ethylimidonium ethosulphate,
- ammonium salts, such as N,N,N-trimethyl-1-docosanaminium chloride (behenetrimonium chloride).

The compositions according to the invention can also contain one or more amphoteric surfactants, such as N-acylamino acids, for instance N-alkylaminoacetates and disodium cocoamphodiacetate, and amine oxides, such as stearamine oxide, or also silicone surfactants, such as dimethicone copolyol phosphates, for instance that sold under the name Pecosil PS 100® by the company Phoenix Chemical.

Water-soluble gelling agent

The composition according to the invention can comprise a water-soluble gelling agent.
The water-soluble gelling agents that can be used in the compositions according to the invention can be chosen from:

homopolymers or copolymers of acrylic acid or methacrylic acid or their salts or their esters and in particular the products sold under the names Versicol F® or Versicol K® by the company Allied Colloid, Ultrahold 8® by the company Ciba-Geigy, or the polyacrylic acids of Synthalen K type,
copolymers of acrylic acid and of acrylamide, sold in the form of their sodium salt, under the names Reten® by the company Hercules, the sodium polymethacrylate sold under the name Darvan No. 7® by the company Vanderbilt, and the sodium salts of poly-hydroxycarboxylic acids sold under the name Hydagen F® by the company Henkel,
polyacrylic acid/alkyl acrylate copolymers of Pemulen type,
AMPS (polyacrylamidomethylpropanesulphonic acid partially neutralized with aqueous ammonia and highly crosslinked), sold by the company Clariant,
AMPS/acrylamide copolymers of Sepigel® or Simulgel® type, sold by the company Seppic, and
copolymers of AMPS/alkyl methacrylates which are polyoxyethylenated (crosslinked or noncrosslinked),
proteins, for instance proteins of plant origin, such as wheat or soya proteins; proteins of animal origin, such as keratins, for example keratin hydrolysates and sulphonic keratins;
cellulose polymers, such as hydroxyethylcellulose, hydroxypropylcellulose, methylcellulose, ethylhydroxyethylcellulose or carboxymethylcellulose, and also quaternized cellulose derivatives;
acrylic polymers or copolymers, such as polyacrylates or polymethacrylates;
vinyl polymers, such as polyvinylpyrrolidones, copolymers of methylvinyl ether and of malic anhydride, the copolymer of vinyl acetate and of crotonic acid, copolymers of vinylpyrrolidone and of vinyl acetate; copolymers of vinylpyrrolidone and of caprolactam; polyvinyl alcohol;
optionally modified polymers of natural origin, such as:

gums arabic, guar gum, xanthan derivatives, karaya gum;
alginates and carrageenans;
glycosaminoglycans, hyaluronic acid and its derivatives;
shellac resin, gum sandarac, dammars, elemis or copals;
deoxyribonucleic acid;
mucopolysaccharides such as chondroitin sulphates, and mixtures thereof.

Some of these water-soluble gelling agents can also act as film-forming polymers.
According to a preferred embodiment, the composition comprises at least one AMPS/acrylamide copolymer.
The water-soluble gelling polymer can be present in the composition according to the invention at a solids content ranging from 0.01% to 60% by weight, preferably from 0.5% to 40% by weight, better still from 1% to 30% by weight, or even from 5% to 20% by weight, relative to the total weight of the composition.

Waxes

The composition according to the invention can comprise, in addition to the compound or the mixture of compounds which confer on said composition a threading nature dmax of greater than or equal to 5 mm, one or more additional waxes. These additional waxes alone do not contribute to the threading nature of the composition.
Use may also be made of waxes provided in the form of small particles having a size, expressed as volume-average "effective" diameter D[4,3], of the order of 0.5 to 30 micrometers, in particular of from 1 to 20 micrometers, and more particularly from 5 to 10 micrometers, subsequently denoted by the expression "microwaxes".
The sizes of the particles can be measured by various techniques. Mention may in particular be made of light scattering techniques (dynamic and static), Coulter counter methods, measurements by rate of sedimentation (related to the size via Stokes' law) and microscopy. These techniques make it possible to measure a particle diameter and, for some of them, a particle size distribution.
As microwaxes which may be used in the compositions according to the invention, mention may in particular be made of carnauba microwaxes, such as that sold under the name MicroCare 350® by the company Micro Powders, synthetic wax microwaxes, such as that sold under the name MicroEase 114S® by the company Micro Powders, microwaxes consisting of a mixture of carnauba wax and of polyethylene wax, such as those sold under the names Micro Care 300® and 310® by the company Micro Powders, microwaxes consisting of a mixture of carnauba wax and of synthetic wax, such as that sold under the name Micro Care 325® by the company Micro Powders, polyethylene microwaxes, such as those sold under the names Micropoly 200®, 220®, 220L® and 250S® by the company Micro Powders, and polytetrafluoroethylene microwaxes, such as those sold under the names Microslip 519® and 519L® by the company Micro Powders.
The composition according to the invention can comprise a content of additional waxes ranging from 0.1% to 30% by weight relative to the total weight of the composition, in particular it can contain from 0.5% to 15% by weight, more particularly from 1% to 10% by weight, thereof.

Oils

The composition according to the invention can also comprise one or more oils or non-aqueous fatty substances which are liquid at ambient temperature (25°C) and atmospheric pressure (760 mmHg).
The oil can be chosen from volatile oils and/or non-volatile oils, and mixtures thereof.
The oil(s) can be present in the composition according to the invention at a content ranging from 0.1% to 95% by weight, preferably from 0.5% to 60% by weight, relative to the total weight of the composition.
For the purpose of the present invention, the term "volatile oil" is intended to mean an oil capable of evaporating on contact with keratin fibres in less than one hour, at ambient temperature and atmospheric pressure. The volatile organic solvent(s) and the volatile oils of the invention are volatile cosmetic oils and organic solvents which are liquid at ambient temperature, and which have a non-zero vapour pressure at ambient temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg), and more particularly ranging from 1.3 Pa to 1300 Pa (0.01 to 10 mmHg).

The term "non-volatile oil" is intended to mean an oil which remains on the keratin fibre at ambient temperature and atmospheric pressure for at least several hours and which has in particular a vapour pressure of less than $10^{-3}$ mmHg (0.13 Pa).

These oils can be hydrocarbon-based oils, silicone oils, fluoro oils, or mixtures thereof.

The term "hydrocarbon-based oil" is intended to mean an oil containing mainly hydrogen and carbon atoms and, optionally, oxygen, nitrogen, sulphur and phosphorus atoms. The volatile hydrocarbon-based oils can be chosen from hydrocarbon-based oils having from 8 to 16 carbon atoms, and in particular branched $C_8$-$C_{16}$ alkanes, such as $C_8$-$C_{16}$ isoalkanes of petroleum origin (also known as isoparaffins), for instance isododecane (also known as 2,2,4,4,6-pentamethylheptane), isodecane or isohexadecane, and, for example, the oils sold under the trade names Isopar or Permetyls, branched $C_8$-$C_{16}$ esters, isohexyl neopentanoate, and mixtures thereof. Other volatile hydrocarbon-based oils, such as petroleum distillates, in particular those sold under the name Shell Solt by the company Shell, can also be used. Preferably, the volatile solvent is chosen from volatile hydrocarbon-based oils having from 8 to 16 carbon atoms and mixtures thereof.

As volatile oils, use may also be made of volatile silicones, such as, for example, volatile linear or cyclic silicone oils, in particular those having a viscosity ≤ 8 centistokes ($8x10^{-6}m^2/s$) and having in particular from 2 to 7 silicon atoms, these silicones optionally comprising alkyl or alkoxy groups having from 1 to 10 carbon atoms. As a volatile silicone oil that can be used in the invention, mention may in particular be made of octamethylcyclotetrasiloxane, decamethylcyclopentasiloxane, dodecamethylcyclohexasiloxane, heptamethylhexyltrisiloxane, heptamethyl-octyltrisiloxane, hexamethyldisiloxane, octamethyltrisiloxane, decamethyltetrasiloxane, dodecamethyl-pentasiloxane, and mixtures thereof.

Mention may also be made of the volatile linear alkyl-trisiloxane oils of general formula (I):

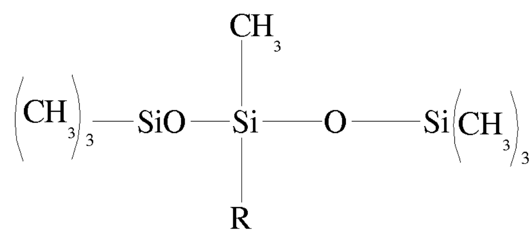

where R represents an alkyl group comprising from 2 to 4 carbon atoms, and one or more hydrogen atoms of which can be substituted with a fluorine or chlorine atom.

Among the oils of general formula (I), mention may be made of:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is respectively a butyl group, a propyl group or an ethyl group.

Use may also be made of volatile fluoro solvents such as nonafluoromethoxybutane or perfluoromethylcyclopentane.

The composition can also comprise at least one non-volatile oil, in particular chosen from non-volatile hydrocarbon-based oils and/or silicone oils and/or fluoro oils.

As non-volatile hydrocarbon-based oil, mention may in particular be made of:

- hydrocarbon-based oils of plant origin, such as triesters of fatty acids and of glycerol, the fatty acids of which can have varied chain lengths from $C_4$ to $C_{24}$, it being possible for the latter to be linear or branched and saturated or unsaturated; these oils are in particular wheatgerm, sunflower, grapeseed, sesame, maize, apricot, castor, shea, avocado, olive, soybean, sweet almond, palm, rapeseed, cotton seed, hazelnut, macadamia, jojoba, alfalfa, poppy, marrow, blackcurrant, evening primrose, millet, barley, quinoa, rye, safflower, candlenut, passion flower or musk rose oil; or else triglycerides of caprylic/capric acids, such as those sold by the company Stearineries Dubois or those sold under the names Miglyol 810, 812 and 818 by the company Dynamit Nobel,
- synthetic ethers having from 10 to 40 carbon atoms;
- linear or branched hydrocarbons of mineral or synthetic origin, such as petroleum jelly, polydecenes, hydrogenated polyisobutene, for instance parleam, squalene, and mixtures thereof;
- synthetic esters, such as the oils of formula R1COOR2 in which R1 represents the residue of a linear or branched fatty acid containing from 1 to 40 carbon atoms and R2 represents a hydrocarbon-based chain, in particular a branched hydrocarbon-based chain, containing from 1 to 40 carbon atoms, provided that R1 + R2 is ≥ 10, for instance

Purcellin oil (cetostearyl octanoate), isopropyl myristate, isopropyl palmitate, $C_{12}$ to $C_{15}$ alkyl benzoate, hexyl laurate, diisopropyl adipate, isononyl isononanoate, 2-ethylhexyl palmitate, isostearyl isostearate, octanoates, decanoates or ricinoleates of alcohols or of polyalcohols, such as propylene glycol dioctanoate; hydroxylated esters, such as isostearyl lactate or diisostearyl malate; and pentaerythritol esters;

- fatty alcohols which are liquid at ambient temperature and which comprise a branched and/or unsaturated carbon chain having from 12 to 26 carbon atoms, such as octyldodecanol, isostearyl alcohol, oleyl alcohol, 2-hexyldecanol, 2-butyloctanol or 2-undecylpentadecanol;
- higher fatty acids, such as oleic acid, linoleic acid or linolenic acid;
- carbonates,
- acetates,
- citrates,
- and mixtures thereof.

The non-volatile silicone oils that can be used in the composition according to the invention can be non-volatile polydimethylsiloxanes (PDMSs), polydimethylsiloxanes comprising pendent alkyl or alkoxy groups and/or alkyl or alkoxy groups at the end of the silicone chain, groups each having from 2 to 24 carbon atoms, phenylated silicones, such as phenyl trimethicones, phenyl dimethicones, phenyl(trimethyl-siloxy)diphenylsiloxanes, diphenyl dimethicones, diphenyl(methyldiphenyl)trisiloxanes or (2-phenylethyl)trimethylsiloxysilicates.

The fluoro oils that can be used in the invention are in particular fluorosilicone oils, fluoro polyethers or fluoro silicones, as described in document EP-A-847752.

Film-forming polymer

The composition according to the invention can comprise, in addition to the compound or the mixture of compounds conferring on the composition a dmax of greater than or equal to 5 mm, at least one film-forming polymer.

The film-forming polymer can be present in the composition according to the invention at a solids (or active material) content ranging from 0.1% to 30% by weight relative to the total weight of the composition, preferably from 0.5% to 20% by weight, and better still from 1% to 15% by weight.

In the present invention, the term "film-forming polymer" is intended to mean a polymer capable of forming, on its own or in the presence of an additional agent which is able to form a film, a macroscopically continuous film which adheres to keratin fibres.

Among the film-forming polymers that can be used in the composition of the present invention, mention may be made of synthetic polymers of radical type or of polycondensate type, polymers of natural origin, and mixtures thereof.

The term "radical film-forming polymer" is intended to mean a polymer obtained by polymerization of monomers possessing unsaturation, in particular ethylenic unsaturation, each monomer being capable of homo-polymerizing (unlike polycondensates).

The film-forming polymers of radical type can in particular be vinyl polymers or copolymers, in particular acrylic polymers.

The film-forming vinyl polymers can result from the polymerization of monomers possessing ethylenic unsaturation having at least one acid group and/or of esters of these acidic monomers and/or of amides of these acidic monomers.

As monomer bearing an acid group, use may be made of unsaturated $\alpha,\beta$-eylenic carboxylic acids, such as acrylic acid, methacrylic acid, crotonic acid, maleic acid or itaconic acid. (Meth)acrylic acid and crotonic acid are preferably used, and more preferably (meth)acrylic acid.

The esters of acidic monomers are advantageously chosen from esters of (meth)acrylic acid (also known as (meth)acrylates), especially alkyl (meth)acrylates, in particular $C_1$-$C_{30}$ alkyl (meth)acrylates, preferably $C_1$-$C_{20}$ alkyl (meth)acrylates, aryl (meth)acrylates, in particular $C_6$-$C_{10}$ aryl (meth)acrylates, and hydroxyalkyl (meth)acrylates, in particular $C_2$-$C_6$ hydroxyalkyl (meth)acrylates.

Among alkyl (meth)acrylates, mention may be made of methyl methacrylate, ethyl methacrylate, butyl methacrylate, isobutyl methacrylate, 2-ethylhexyl methacrylate, lauryl methacrylate and cyclohexyl methacrylate.

Among hydroxyalkyl (meth)acrylates, mention may be made of hydroxyethyl acrylate, 2-hydroxypropyl acrylate, hydroxyethyl methacrylate and 2-hydroxypropyl methacrylate.

Among aryl (meth)acrylates, mention may be made of benzyl acrylate and phenyl acrylate.

The esters of (meth)acrylic acid which are particularly preferred are alkyl (meth)acrylates.

According to the present invention, the alkyl group of the esters can be fluorinated or perfluorinated, i.e. a portion or all of the hydrogen atoms of the alkyl group are substituted with fluorine atoms.

As amides of the acidic monomers, mention may, for example, be made of (meth)acrylamides, especially N-alkyl(meth)acrylamides, in particular N- ($C_2$-$C_{12}$ alkyl)(meth)acrylamides. Among the N-alkyl(meth)-acrylamides, mention may be made of N-ethylacrylamide, N-t-butylacrylamide, N-t-octylacrylamide and N-undecyl-acrylamide.

The film-forming vinyl polymers can also result from the homopolymerization or from the copolymerization of monomers chosen from vinyl esters and styrene monomers. In particular, these monomers can be polymerized with acidic monomers and/or their esters and/or their amides, such as those mentioned above.

As an example of vinyl esters, mention may be made of vinyl acetate, vinyl neodecanoate, vinyl pivalate, vinylbenzoate and vinyl t-butylbenzoate.

As styrene monomers, mention may be made of styrene and alpha-methylstyrene.

Among the film-forming polycondensates, mention may be made of polyurethanes, polyesters, polyesteramides, polyamides, epoxy ester resins and polyureas.

The polyurethanes can be chosen from anionic, cationic, non-ionic or amphoteric polyurethanes, polyurethane-acrylics, polyurethane-polyvinylpyrrolidones, polyester-polyurethanes, polyether-polyurethanes, polyureas, polyurea-polyurethanes, and blends thereof.

The polyesters can be obtained, in a known manner, by polycondensation of dicarboxylic acids with polyols, in particular diols, as described above for the sulphopolyester.

The optionally modified polymers of natural origin can be chosen from shellac resin, gum sandarac, dammars, elemis, copals, cellulose-based polymers, and mixtures thereof.

According to a first embodiment of the composition according to the invention, the film-forming polymer can be a water-soluble polymer and can be present in an aqueous phase of the composition; the polymer is therefore solubilized in the aqueous phase of the composition.

According to another variant embodiment of the composition according to the invention, the film-forming polymer can be a polymer solubilized in a liquid fatty phase comprising organic solvents or oils such as those described above (the film-forming polymer is then referred to as a liposoluble polymer). Preferably, the liquid fatty phase comprises a volatile oil, optionally as a mixture with a non-volatile oil, it being possible for the oils to be chosen from the oils mentioned above.

By way of example of a liposoluble polymer, mention may be made of copolymers of a vinyl ester (the vinyl group being directly linked to the oxygen atom of the ester group and the vinyl ester having a linear or branched, saturated hydrocarbon-based radical containing from 1 to 19 carbon atoms, attached to the carbonyl of the ester group) and of at least one other monomer, which can be a vinyl ester (different from the vinyl ester already present), an $\alpha$-olefin (having from 8 to 28 carbon atoms), an alkyl vinyl ether (the alkyl group of which contains from 2 to 18 carbon atoms), or an allyl or methallyl ester (having a linear or branched, saturated hydrocarbon-based radical containing from 1 to 19 carbon atoms, attached to the carbonyl of the ester group).

These copolymers can be crosslinked using crosslinking agents which can be either of the vinyl type or of the allyl or methallyl type, such as tetraallyloxyethane, divinylbenzene, divinyl octanedioate, divinyl dodecanedioate or divinyl octadecanedioate.

As examples of these polymers, mention may be made of the copolymers: vinyl acetate/allyl stearate, vinyl acetate/vinyl laurate, vinyl acetate/vinyl stearate, vinyl acetate/octadecene, vinyl acetate/octadecyl vinyl ether, vinyl propionate/allyl laurate, vinyl propionate/vinyl laurate, vinyl stearate/1-octadecene, vinyl acetate/1-dodecene, vinyl stearate/ethyl vinyl ether, vinyl propionate/cetyl vinyl ether, vinyl stearate/allyl acetate, vinyl 2,2-dimethyloctanoate/- vinyl laurate, allyl 2,2-dimethylpentanoate/vinyl laurate, vinyl dimethylpropionate/vinyl stearate, allyl dimethylpropionate/vinyl stearate, vinyl propionate/- vinyl stearate, crosslinked with 0.2% of divinylbenzene, vinyl dimethylpropionate/vinyl laurate, crosslinked with 0.2% of divinylbenzene, vinyl acetate/octadecyl vinyl ether, crosslinked with 0.2% of tetraallyloxyethane, vinyl acetate/allyl stearate, crosslinked with 0.2% of divinylbenzene, vinyl acetate/1-octadecene crosslinked with 0.2% of divinylbenzene, and allyl propionate/allyl stearate crosslinked with 0.2% of divinylbenzene.

As liposoluble film-forming polymers, mention may also be made of liposoluble copolymers, and in particular those resulting from copolymerization of vinyl esters having from 9 to 22 carbon atoms or from alkyl acrylates or alkyl methacrylates, the alkyl radicals having from 10 to 20 carbon atoms.

Such liposoluble copolymers can be chosen from copolymers of poly(vinyl stearate), of poly(vinyl stearate) crosslinked with divinylbenzene, of diallyl ether or of diallyl phthalate, copolymers of poly-(stearyl (meth)acrylate), of poly(vinyl laurate), of poly(lauryl (meth)acrylate), it being possible for these poly(meth)acrylates to be crosslinked using ethylene glycol dimethacrylate or tetraethylene glycol dimethacrylate.

The liposoluble copolymers defined above are known and are in particular described in application FR-A-2232303; they can have a weight-average molecular weight ranging from 2000 to 500 000, and preferably from 4000 to 200 000.

As liposoluble film-forming polymers that can be used in the invention, mention may also be made of polyalkylenes, and in particular $C_2$-$C_{20}$ alkene copolymers, such as polybutene, alkylcelluloses with a linear or branched saturated or unsaturated $C_1$ to $C_8$ alkyl radical, such as ethylcellulose or propylcellulose, vinylpyrrolidone (VP) copolymers, and in particular copolymers of vinylpyrrolidone and of a $C_2$ to $C_{40}$, and better still $C_3$ to $C_{20}$, alkene. By way of example of a VP copolymer that can be used in the invention, mention may be made of the copolymer of VP/vinyl acetate, VP/ethyl methacrylate, butylated polyvinylpyrrolidone (PVP), VP/ethyl methacrylate/methacrylic acid, VP/eicosene, VP/hexadecene, VP/triacontene, VP/styrene, VP/acrylic acid/lauryl methacrylate.

Mention may also be made of silicone resins, generally soluble or swellable in silicone oils, which are crosslinked polyorganosiloxane polymers. The nomenclature of silicone resins is known as "MDTQ", the resin being described according to the various siloxane monomer units which it comprises, each of the letters "MDTQ" characterizing one type of unit.

By way of examples of commercially available poly- methylsilsesquioxane resins, mention may be made of those which are sold:

by the company Wacker under the reference resin MK, such as Belsil PMS MK;
by the company Shin-Etsu under the references KR-220L.

As siloxysilicate resins, mention may be made of trimethylsiloxysilicate (TMS) resins, such as those sold under the reference SR1000 by the company General Electric or under the reference TMS 803 by the company Wacker. Mention may also be made of the trimethylsiloxysilicate resins sold in a solvent, such as cyclomethicone, sold under the name "KF-7312J" by the company Shin-Etsu, or "DC 749" or "DC 593" by the company Dow Corning.

Mention may also be made of copolymers of silicone resins, such as those mentioned above with polydimethylsiloxanes, for instance the pressure-sensitive adhesive copolymers sold by the company Dow Corning under the reference BIO-PSA and described in document US 5 162 410, or the silicone copolymers derived from the reaction of a silicone resin, such as those described above, and of a diorganosiloxane, as described in document WO 2004/073626.

The film-forming polymer can also be present in the composition in the form of particles in dispersion in an aqueous phase or in a non-aqueous solvent phase, generally known as latex or pseudolatex. The techniques for preparing these dispersions are well known to those skilled in the art.

As aqueous film-forming polymer dispersion, use may be made of the acrylic dispersions sold under the names Neocryl XK-90®, Neocryl A-1070®, Neocryl A-1090®, Neocryl BT-62®, Neocryl A-1079® and Neocryl A-523® by the company Avecia-Neoresins, Dow Latex 432® by the company Dow Chemical, Daitosol 5000 AD® or Daitosol 5000 SJ® by the company Daito Kasey Kogyo; Syntran 5760® by the company Interpolymer, Allianz OPT by the company Rohm & Haas, the aqueous dispersions of acrylic or styrene/acrylic polymers sold under the trade name Joncryl® by the company Johnson Polymer, or else the aqueous dispersions of polyurethane sold under the names Neorez R-981®' and Neorez R-974® by the company Avecia-Neoresins, Avalure UR-405®, Avalure UR-410®, Avalure UR-425®, Avalure UR-450®, Sancure 875®, Sancure 861®, Sancure 878® and Sancure 2060® by the company Goodrich, Impranil 85® by the company Bayer, Aquamere H-1511® by the company Hydromer; the sulphopolyesters sold under the trade name Eastman AQ® by the company Eastman Chemical Products, vinyl dispersions such as Mexomère PAM® from the company Chimex and blends thereof.

As examples of non-aqueous dispersions of film-forming polymer, mention may be made of acrylic dispersions in isododecane, such as Mexomère PAP® from the company Chimex, dispersions of particles of a grafted ethylenic polymer, preferably grafted acrylic polymer, in a liquid fatty phase, the ethylenic polymer being advantageously dispersed in the absence of additional stabilizer at the surface of the particles, as described, in particular, in document WO 04/055081.

The composition according to the invention can comprise a plasticizer which promotes the formation of a film with the film-forming polymer. Such a plasticizer can be chosen from all the compounds known to those skilled in the art to be capable of performing the desired function.

The composition can also comprise ingredients commonly used in cosmetics, such as lipophilic gelling agents, dyestuffs, fillers, fibres, and mixtures thereof.

Dyestuff

The composition according to the invention can also comprise at least one dyestuff, such as pulverulent materials, liposoluble dyes or water-soluble dyes.

The pulverulent dyestuffs can be chosen from pigments and pearlescent agents.

The pigments may be white or coloured, inorganic and/or organic, and coated or uncoated. Among the inorganic pigments, mention may be made of titanium dioxide, optionally surface-treated, zirconium oxide, zinc oxide or cerium oxide, and also iron or chromium oxides, manganese violet, ultramarine blue, chromium hydrate and ferric blue. Among the organic pigments, mention may be made of carbon black, pigments of D & C type, and lakes based on cochineal carmine, barium, strontium, calcium or aluminium.

The pearlescent agents can be chosen from white pearlescent agents, such as mica coated with titanium oxide or with bismuth oxychloride, coloured pearlescent pigments, such as titanium oxide-coated mica with iron oxides, titanium oxide-coated mica with in particular ferric blue or chromium oxide, or titanium oxide-coated mica with an organic pigment of the abovementioned type, and pearlescent agents based on bismuth oxychloride.

The liposoluble dyes are, for example, Sudan red, D&C Red 17, D&C Green 6, β-carotene, soybean oil, Sudan brown, D&C Yellow 11, D&C Violet 2, D&C Orange 5, quinoline yellow or annatto.

These dyestuffs can be present at a content ranging from 0.01% to 30% by weight relative to the total weight of the composition.

The composition used in the present invention can comprise substances which have a non-zero magnetic susceptibility, also called "magnetic bodies", which can be in various forms.

Thus, according to one embodiment, a subject of the invention is a cosmetic process for making up or for the non-therapeutic care of keratin fibres, comprising the following steps:

a) applying to the keratin fibres, preferably in the absence of a magnetic field, a cosmetic composition comprising magnetic bodies and at least one compound or mixture of compounds, which, when the composition is brought to a temperature of greater than or equal to 40°C, confers on said composition a threading nature dmax of greater than or equal to 5 mm,

b) bringing said composition, prior to, simultaneously with or subsequent to its application, to a temperature of greater than or equal to 40°C,

c) at least partially subjecting said composition to a magnetic field so as to shift and/or modify the orientation of at least some of the substances with non-zero magnetic susceptibility,

the order of steps b) and c) being unimportant. Preferably, the magnetic field is applied during application or after application of the composition.

The application of the composition in the absence of a magnetic field assumes the use of an applicator which is non-magnetic or weakly magnetic at the time of application, and which does not interact with the composition to the point of significantly modifying the way in which it is applied. The applicator can thus advantageously be non-magnetic and entirely conventional.

The magnetic field is, for example, generated by a magnetic device separate from the applicator or solidly joined to the latter but sufficiently remote from the application member so as not to significantly interact with the composition during application.

Such a process makes it possible to obtain a reinforcement of the eyelash lengthening effect, and/or an eyelash curling effect and/or an improvement in eyelash separation.

The magnetic field can be generated in such a way as to subject the composition present on the eyelashes to field lines substantially parallel to the eyelashes, for the purpose of lengthening and/or separating the eyelashes. The magnetic field can, for example, be generated by a magnet or an electromagnet which has a polar axis substantially along the axis of the eyelashes. Such a magnetic field is favourable to a displacement of the composition in the direction of the extension of the eyelash.

The expression "magnetic bodies" should not be understood in a limiting manner, and covers particles, fibres or agglomerates of particles and/or of fibres, of all shapes, having a non-zero magnetic susceptibility.

The concentration of magnetic bodies in the composition is, for example, between approximately 0.05% and approximately 50% by mass, in particular between approximately 0.1% and approximately 40% by mass, better still between approximately 1% and approximately 30% by mass.

The composition applied can comprise magnetic fibres or other aspherical bodies, such as chains of particles or of fibres.

Preferably, the magnetic bodies do not exhibit any persistent magnetization in the absence of a magnetic field.

The magnetic bodies can comprise any magnetic material which exhibits sensitivity to the lines of a magnetic field, irrespective of whether this field is produced by a magnet which is permanent or derived from an induction, this material being, for example, chosen from nickel, cobalt, iron, their alloys and oxides, in particular $Fe_3O_4$, and also gadolinium, terbium, dysprosium, erbium, and their alloys and oxides. The magnetic material may be of "soft" or "hard" type. The magnetic material may in particular be soft iron, which has a high susceptibility and can facilitate the obtaining of a thickness-extending effect.

The magnetic bodies may or may not have a multilayer structure, comprising at least one layer of a magnetic material, such as, for example, iron, nickel, cobalt, and their alloys and oxides, in particular $Fe_3O_4$.

The magnetic bodies are preferably aspherical, having, for example, an elongated shape. Thus, when these bodies are subjected to the magnetic field, they tend to orient themselves with their longitudinal axis aligned along the field lines, and undergo a change in orientation which is reflected by a change in appearance of the composition. When the magnetic bodies are substantially spherical particles, their appearance is preferably non-homogeneous, such that a change in orientation induces a change in appearance.

The size of the bodies, irrespective of their shape, is, for example, between 1 nm and 10 mm, better still between 10 nm and 5 mm, even better still between 100 nm and 1 mm, for example between 0.5 μm and 300 μm or 1 μm and 150 μm. The size is that given by the statistical distribution with respect to half the population, referred to as D50.

When the bodies are particles which do not have an elongated shape or which have an elongated shape with quite a low shape factor, the size of the particles is, for example, less than 1 mm.

The magnetic bodies are, for example, magnetic pigments.

Magnetic pigments

Pigments that are most particularly suitable are pearlescent agents comprising iron oxide $Fe_3o_4$. Pigments having magnetic properties are, for example, those sold under the trade names Colorona Blackstar Blue, Colorona Blackstar Green, Colorona Blackstar Gold, Colorona Blackstar Red, Cloisonne Nu Antique Super Green, Microna Matte Black (17437), Mica Black (17260), Colorona Patina Silver (17289) and Colorona Patina Gold (117288) from the company Merck, or else Flamenco Twilight Red, Flamenco Twilight Green, Flamenco Twilight Gold, Flamenco Twilight Blue, Timica Nu Antique Silver 110 AB, Timica Nu Antique Gold 212 GB, Timica Nu-Antique Copper 340 AB, Timica Nu Antique Bronze 240 AB, Cloisonne Nu Antique Green 828 CB, Cloisonne Nu Antique Blue 626 CB, Gemtone Moonstone G 004, Cloisonne Nu Antique Red 424 CB, Chroma-Lite Black (4498), Cloisonne Nu Antique Rouge Flambe (code 440 XB), Cloisonne Nu Antique Bronze (240 XB), Cloisonne Nu Antique Gold (222 CB) and Cloisonne Nu Antique Copper (340 XB) from the company Engelhard.

By way of further example of a magnetic pigment that may be part of the formulation of the composition, mention may be made of particles of black iron oxide, for example those sold under the name Sicovit noir E172 by the company BASF.

The magnetic pigments can also comprise metal iron, in particular passivated soft iron, for example obtained from carbonyl iron using the process described in US patent 6,589,331, the content of which is incorporated by way of reference. These particles can comprise a surface layer of an oxide.

Particles based on soft iron are sold in particular under the name Stapa® WM Iron VP 041040 by the company Eckart.

Magnetic fibres

The term "fibres" denotes bodies which are generally elongated, having, for example, a shape factor ranging from 3.5 to 2500 or from 5 to 500, for example from 5 to 150. The shape factor is defined by the ratio L/D, where L is the length of the fibre and D the diameter of the circle into which the largest transverse section of the fibre falls.

The transverse section of the fibres can, for example, fall into a circle of diameter ranging from 2 nm to 500 $\mu$m, for example ranging from 100 nm to 100 $\mu$m, or even from 1 $\mu$m to 50 $\mu$m. The fibres can have, for example, a length ranging from 1 $\mu$m to 10 mm, for example from 0.1 mm to 5 mm, or even from 0.3 mm to 3.5 mm.

The fibres can have a mass ranging, for example, from 0.15 to 30 denier (mass in grams per 9 km of thread), for example from 0.18 to 18 denier.

The fibres can have any transverse sectional shape, for example circular or polygonal, in particular square, hexagonal or octagonal.

The composition can comprise fibres which are solid or hollow, and independent or bound to one another, for example braided.

The composition can comprise fibres having ends which have been blunted and/or rounded, for example by polishing.

It is possible for the shape of the fibres not to be substantially modified when the latter are introduced into the composition, said fibres being, for example, initially rectilinear and sufficiently rigid to conserve their shape. In a variant, the fibres can have a flexibility which allows them to substantially change shape in the composition.

The fibres can comprise a non-zero content, that can range up to 100%, of a magnetic material chosen from soft magnetic materials, hard magnetic materials, in particular based on iron, on zinc, on nickel, on cobalt or on manganese and their alloys and oxides, in particular $Fe_3O_4$, rare earth metals, barium sulfate, iron-silicon alloys, optionally charged with molybdenum, $Cu_2MnAl$, MnBi, or a mixture thereof, this list not being limiting.

When the composition comprises fibres containing magnetic particles, the latter can be present, for example, at least at the surface of the fibre, or even only at the surface of the fibres, or only inside the fibre, or else can be dispersed within the fibre in a substantially homogeneous manner.

The fibres can comprise, for example, a non-magnetic core with a plurality of magnetic particles at its surface.

The fibres can also comprise a synthetic matrix containing a plurality of magnetic grains dispersed within it.

Where appropriate, a synthetic material loaded with magnetic particles can itself be coated with a non-magnetic shell. Such a shell constitutes, for example, a barrier which insulates the magnetic material(s) from the ambient medium and/or can provide colour. The fibres can comprise a monolithic magnetic core and can be coated with a non-magnetic shell, or vice versa. The composition can comprise fibres produced by extrusion or coextrusion of one or more polymeric materials, in particular thermoplastic and/or elastomeric materials. One of the extruded materials can contain a load of

dispersed magnetic particles. The fibre can comprise a synthetic material chosen from polyamides, PET, acetates, polyolefins, in particular PE or PP, PVC, polyester bloc amide, plasticized Rilsan®, elastomers, in particular polyester elastomers, PE elastomers, silicone elastomers, nitrile elastomers or a mixture of these materials, this list not being limiting.

The composition can contain composite fibres comprising a magnetic core coated at least partially with at least one synthetic or natural amagnetic material. The coating of the magnetic core can be carried out, for example, by coextrusion, around the core, of a shell made of a non-magnetic material.

The coating of the core can also be carried out in another way, for example by *in situ* polymerization.

The core can be monolithic or can comprise a load made up of magnetic grains dispersed in a matrix.

The composition can also contain composite fibres obtained by coating a synthetic or natural amagnetic core with a synthetic material loaded with magnetic particles, the core being composed, for example, of a wood fibre, a rayon fibre, a polyamide fibre, a fibre of a plant material, a polyolefin fibre, in particular a polyethylene fibre, a Nylon® fibre, a polyimideamide fibre or an aramide fibre, this list not being limiting.

The composition can also comprise magnetic composite particles, in particular a magnetic latex.

Magnetic composite particles

A magnetic composite particle is a composite material consisting of an organic or inorganic matrix and of magnetic grains. The magnetic composite particles can thus comprise, at their surface and/or within them, grains of a magnetic material. The composite particles can consist of a magnetic core coated with an organic or inorganic matrix, or vice versa.

The magnetic composite particles comprise, for example, one of the abovementioned magnetic materials.

The size of the magnetic composite particles is, for example, between 1 nm and 1 mm, better still between 100 nm and 500 $\mu$m, better still between 500 nm and 100 $\mu$m. The term "size" denotes the size given by the statistical particle size distribution with respect to half the population, referred to as D50.

The thesis by C. Goubault, 23 March 2004, incorporated herein by way of reference, recalls, in Chapter 1, the prior art regarding magnetic composite particles and draws up a list of preparation processes that can be used for preparing magnetic composite particles, i.e. separate synthesis of the magnetic grains and of the matrix, synthesis of the magnetic grains in contact with the matrix or synthesis of the matrix in the presence of the magnetic grains.

The company Kisker sells composite magnetic particles comprising an inorganic matrix composed of silica. The companies Dynal, Seradyn, Estapor and Ademtech propose composite magnetic particles comprising an organic matrix, which can also be used in the invention.

More particularly, the company Estapor sells, under the reference M1-070/60, magnetic latices consisting of grains of ferrite uniformly distributed in a polystyrene matrix, this latex comprising 65% of iron oxide, the average diameter of the polystyrene particles being 890 nm and the solids content by mass being 10%.

Ferrofluid

The composition can comprise a ferrofluid, i.e. a stable colloidal suspension of magnetic particles, in particular of magnetic nanoparticles.

The particles, which are, for example, of the order of a few tens of nanometres in size, are dispersed in a solvent (water, oil, organic solvent), either by means of a surfactant or a dispersing agent, or by electrostatic interactions.

The ferrofluids are, for example, prepared by milling ferrites or other magnetic particles until nanoparticles are obtained, which are then dispersed in a fluid containing a surfactant, which adsorbs onto the particles and stabilizes them, or by precipitation, in a basic medium, of a solution of metal ions.

Each particle of the ferrofluid has a magnetic moment determined by the size of the particle and by the nature of the magnetic material.

Under the action of a magnetic field, the magnetic moments of the particles tend to align themselves along the field lines, with the appearance of a non-zero magnetization in the liquid. If the field is withdrawn, there is no hysteresis and the magnetization is withdrawn.

Beyond a threshold field value, macroscopic changes can also be brought about in the liquid, for example the appearance of peaks or a modification of the rheological properties.

The name "ferrofluid" also encompasses an emulsion of droplets of ferrofluid in a solvent. Each drop then contains colloidal magnetic particles in a stable suspension. This makes it possible to have a ferrofluid in any type of solvent. The size of the magnetic particles in suspension in the ferrofluid is, for example, between 1 nm and 10 $\mu$m, better still between 1 nm and 1 $\mu$m, even better still between 1 nm and 100 nm. The term "size" denotes the size given by the statistical particle size distribution with respect to half the population, referred to as D50.

Mention may in particular be made of the ferrofluids sold by the company Liquids Research Ltd under the

references:

- WHKS1S9 (A, B or C), which is a water-based ferrofluid comprising magnetite ($Fe_3O_4$), having particles 10 nm in diameter.
- WHJS1 (A, B or C), which is an isoparaffin-based ferrofluid comprising particles of magnetite ($Fe_3O_4$) 10 nm in diameter.
- BKS25 dextran, which is a water-based ferrofluid stabilized with dextran, comprising particles of magnetite ($Fe_3O_4$) 9 nm in diameter.

Magnetic particle and/or fibre chains

The composition can also comprise, as magnetic bodies, magnetic particle and/or fibre chains.

The composition can thus comprise agglomerates of particles or fibres whose largest dimension, for example the length, is, for example, between 1 nm and 10 mm, for example between 10 nm and 5 mm, or between 100 nm and 1 mm, or else between 0.5 μm and 3.5 mm, for example between 1 μm and 150 μm. The size denotes that given by the statistical particle size distribution with respect to half the population, referred to as D50.

Chains of magnetic particles can be obtained, for example, by assembling colloidal magnetic particles, as described in the publications "Permanently linked monodisperse paramagnetic chains", E.M. Furst, C. Suzuki, M. Fermigier, A.P. Gast, Langmuir, 14, 7334-7336 (1998), "Suspensions de particules magnétiques" [Suspensions of magnetic particles], M. Fermigier, Y. Grasselli, Bulletin de la SFP [SFP bulletin] (105) July 96, and "Flexible magnetic filaments as micromechanical sensors", C. Goubault, P. Jop, M. Fermigier, J. Baudry, E. Bertrand, J. Bibette, Phys. Rev. Lett., 91, 26, 260802-1 to 260802-4 (2003), the contents of which are incorporated by way of reference.

These articles describe in particular how to proceed in order to obtain chains of magnetic latex particles which comprise a polystyrene matrix containing grains of iron oxide and which are surface-functionalized, said particles being permanently attached to one another following a chemical reaction, in particular covalent bonds between the surfaces of the adjacent particles; they also describe a process for obtaining chains of droplets of an emulsion of ferrofluids, attached to one another by interactions of a physical nature. The length and the diameter of the permanent chains thus obtained can be controlled. Such magnetic chains constitute anisotropic magnetic objects which can be oriented and moved under the effect of a magnetic field. The sizes of the magnetic chains can correspond to the same conditions as the magnetic fibres.

Magnetic particles and application devices are in particular described in WO 06/037900.

Fillers

The composition according to the invention can also comprise at least one filler.

The fillers can be chosen from those well known to those skilled in the art and commonly used in cosmetic compositions. The fillers can be inorganic or organic, and lamellar or spherical. Mention may be made of talc, mica, silica, kaolin, powders of polyamide, such as Nylon®, sold under the name Orgasol® by the company Atochem, of poly-β-alanine and of polyethylene, powders of tetrafluoroethylene polymers such as Teflon®, lauroyl lysine, starch, boron nitride, expanded hollow polymer microspheres such as those of polyvinylidene chloride/acrylonitrile, for instance those sold under the name Expancel® by the company Nobel Industrie, acrylic powders such as those sold under the name Polytrap® by the company Dow Corning, particles of poly(methyl methacrylate) and silicone resin microbeads (Tospearls® from Toshiba, for example), precipitated calcium carbonate, magnesium carbonate and basic magnesium carbonate, hydroxyapatite, hollow silica microspheres (Silica Beads® from Maprecos), glass or ceramic microcapsules, metal soaps derived from organic carboxylic acids having from 8 to 22 carbon atoms, and in particular from 12 to 18 carbon atoms, for example zinc stearate, magnesium stearate or lithium stearate, zinc laurate or magnesium myristate.

Use may also be made of a compound capable of swelling in reaction to heat, and in particular heat-expandable particles such as nonexpanded microspheres of vinylidene chloride/acrylonitrile/methyl methacrylate copolymer or of copolymer of acrylonitrile homopolymer, for instance those sold respectively under the references Expancel® 820 DU 40 and Expancel® 007WU by the company Akzo Nobel.

The fillers can represent from 0.1% to 25%, in particular from 1% to 20% by weight, relative to the total weight of the composition.

The composition of the invention can also comprise any additive normally used in cosmetics, such as antioxidants, preserving agents, fibres, fragrances, neutralizing agents, gelling agents, thickeners, vitamins, coalescence agents, plasticizers, and mixtures thereof.

Fibres

The composition according to the invention can also comprise, in addition to the compound or the mixture of compounds which confer on said composition a threading nature dmax of greater than or equal to 5 mm, fibres referred to as additional fibres. These additional fibres alone do not contribute the threading nature of the composition.
The additional fibres can be present in the composition according to the invention at a content ranging from 0.01% to 10% by weight, relative to the total weight of the composition, in particular from 0.1% to 5% by weight, and more particularly from 0.3% to 3% by weight.

Cosmetic active agents

As cosmetic active agents that can be used in the compositions according to the invention, mention may in particular be made of antioxidants, preserving agents, fragrances, neutralizing agents, emollients, moisturizers, vitamins and screening agents, in particular sunscreens.
Of course, those skilled in the art will take care to select the optional supplementary additives and/or the amount thereof in such a way that the advantageous properties of the composition according to the invention are not, or are not substantially, impaired by the addition envisaged.
The composition can be in solid, semi-solid or liquid form.
The composition can in particular be in the form of a suspension, a dispersion, a solution, a gel or an emulsion, in particular an oil-in-water (O/W), wax-in-water or water-in-oil (W/O), or multiple (W/O/W or polyol/O/W or O/W/O) emulsion, or in the form of a cream, a paste, a foam, a dispersion of vesicles, in particular of ionic or non-ionic lipids, a two-phase or multiphase lotion, a spray, a powder or a paste, in particular a supple paste. Each composition is preferably a leave-on composition.
The composition according to the invention can be produced by the known processes, generally used in the cosmetics field.
In the process of the invention, the composition is generally heated to a temperature of greater than or equal to 40°C, especially greater than or equal to 45°C, in particular greater than or equal to 50°C.
Obviously, the heating temperature depends in particular on the temperature that the treated support is capable of withstanding.
According to one embodiment, the composition is in solid form.
According to a first embodiment of the process according to the invention, the composition is solid and is heated prior to its application, it being possible for the heating means used to be the applicator itself. Thus, in the case of a mascara, the composition can be applied using a heating applicator such as a heating brush.
According to another embodiment of the process according to the invention, the composition is heated at the time of its application. In such a case, the heating means used is generally the applicator itself. Thus, in the case of a mascara, the composition can be applied using a heating brush.
According to a second embodiment, the composition is heated subsequent to its application. According to a first variant, the composition can be heated using means not specifically intended for heating, for example a part which is occasionally hot. According to a second variant of this embodiment, the composition can be heated using a means specifically dedicated to the heating. It can in particular be a means which propels hot air, such as a hair dryer or a heating device, for example as described below.
According to one embodiment, the composition according to the invention is in the form of a powder or of a pulverulent mass. This composition can be applied to the keratin fibres using an application device comprising a heating support, the composition being contained in an applicator end piece having a shape suitable for it to be push-fitted onto the heating support, or the composition being contained in a container in which the heating support can be immersed in order to load it up with composition.
The composition according to the invention can be packaged in a packaging and application assembly comprising:

i) a reservoir comprising said composition,
ii) a device for applying the composition, and
iii) heating means.

According to one embodiment, the heating means are made up of a device which is separate from the application device or member, the assembly being configured in the form of a packaging and application device also comprising a container containing a composition in accordance with the invention. Such a device can be packaged in packaging of the blister pack type. The heating means may be of the type such as those described in patents US 6 009 884 or US 5 853 010. Other devices configured in the form of heating tongs (in the case of eyelashes) can also be used. Such devices

are described in particular in patent US 6 220 252.

According to one embodiment, the application device or member comprises means for heating the composition; in particular, the heating means associated with the application device are arranged so as not to substantially heat at least a part of the shaft.

The kit 1 described in Figure 1 comprises a mascara packaging and application assembly 100 and a heating device 50, separate from the packaging and application assembly.

The two devices 100 and 50 can be sold together in the same packaging, of blister pack type. The unit 100 containing the product can be sold separately.

The packaging and application assembly 100 comprises a container 2, comprising the composition according to the invention, on which is mounted a threaded collar 3, one free edge of which delimits an opening 4. In the opening 4 is mounted a draining member 5. The assembly 100 also comprises an application device 10 comprising a stopper 11 solidly fastened to a shaft 13, one end of which comprises an applicator 12, generally configured in the form of an arrangement of fibres held between the two branches of a twisted iron wire. An inner surface of the stopper 11 is threaded so as to engage with the threading of the neck 3. Thus, when the applicator 12 and the shaft 13 are inside the container 2, the threading of the stopper 11 engages with the threading of the neck 3 in such a way that the stopper sealably closes the opening 4 of the container. Such packaging and application assemblies are well known.

The heating device 50 is in accordance with that described in patent US 6 009 884. It mainly comprises a gripping portion 51 and a lid 52. A battery is placed inside the gripping portion 51 and is connected to a heating wire 53 configured in the form of a coil arranged on a shaft 54. A switch 55 allows the device to be switched on and off. An LED 56, when it changes colour, indicates that the device is at the required temperature, and is thus ready for use.

The power supply of the heating part via the battery is 12 V. The power dissipated is approximately 1 watt. The heating wire 53 may be made of nickel/chromium alloy.

In the embodiment of Figure 2, the applicator 12 consists of a metal cylinder, at least a part of the periphery of which is striated perpendicular to its longitudinal axis. The striated cylinder is attached, in particular by bonding, to the end of the shaft 13. A heating resistance 53, which extends over substantially the entire length of the applicator 12, is arranged so that it is diametrically opposed relative to the striated part. The heating resistance 53 can be placed in a groove made longitudinally in the surface of the cylinder.

Thus, the heating resistance 53 heats the composition present on the striated cylinder, the striated zone of the latter being used for the application per se of the product onto the eyelashes, and also for separating them.

According to one embodiment, the mascara is applied cold, in a conventional manner, to the eyelashes by means of a brush 12 and then heated after application. The user engages the heating part 53 of the device 50 with the eyelashes in such a way as to bring the deposit of product to the threading temperature of the composition, and then drawn by means of the heating device formed on the eyelashes so as to create threads along the extension of the eyelashes.

On cooling, the threads are set along the extension of the eyelashes, allowing a lengthening effect to be obtained.

According to another embodiment, the mascara is in solid form and is used with a heating device 50 alone. It is brought into contact with the heating part 53 of the device 50 and then heated so as to bring the deposit of product to the threading temperature of the composition. The user then engages the heating part 53 of the device with the eyelashes and then draws, by means of the device, the deposit formed on the eyelashes so as to create threads along the extension of the eyelashes.

The examples below are presented by way of non-limiting illustration of the invention. Unless otherwise indicated, the amounts indicated are expressed as percentage by mass.

**Example 1: Mascara**

A mascara having the following composition was prepared:

| Phase A | |
|---|---|
| Coolbind 34-1300 from National Starch | 43% |
| **Phase B** | |
| Octyldodecanol | 6.18% |
| Black iron oxide | 1.7% |

(continued)

| Phase C | |
|---|---|
| Potassium cetyl phosphate at 79.5% in a mixture with phosphoric acid, cetyl alcohol and water (Arlatone MAP 160K from Givaudan) | 2.54% |
| PEG-30 glyceryl stearate | 6.18% |
| Acrylamide/sodium acyloyldimethyltaurate copolymer (Simulgel 600, Seppic) | 0.68% AM* |
| Preserving agents | qs |
| Water | qs 100% |
| *AM = active material | |

Procedure:

This mascara is prepared in a twin-screw mixer-extruder (of the prism type from Thermo Electron Corporation, England) comprising 6 independent barrels, each making it possible to introduce a new phase and to fix the temperature. They are numbered from 1 to 6 from the inlet to the outlet of the product. The flow rate is 2 kg/h (1000 rpm).

The ingredients of phases A and B are introduced respectively into the first and second barrels, in which they are heated to 150°C.

The ingredients of phase C, preheated to 80°C, are introduced into the second barrel, in which the three phases are mixed under hot conditions.

The composition is then cooled so as to be recovered at ambient temperature.

This mascara has a threading nature under hot conditions, with threads of 23 mm on average. The threads obtained are fine, rigid and black. They are sufficiently rigid so as not to bend under their own weight, remaining vertical.

**Example 2**

A mascara having the following composition was prepared:

| | |
|---|---|
| Coolbind 34-1300 from National Starch | 20% |
| Octyldodecanol | 8.67% |
| Potassium cetyl phosphate at 79.5% in a mixture with phosphoric acid, cetyl alcohol and water (Arlatone MAP 160K from Givaudan) | 3.57% |
| PEG-30 glyceryl stearate | 8.67% |
| Copolymer of acrylamide/AMPS Na in isohexadecane with polysorbate 80 (Simulgel 600® from Seppic) | 0.96% AM |
| Preserving agents | qs |
| Black iron oxide | 2.4% |
| Water | qs 100% |

Procedure:

The ethylene vinyl acetate, the octyldodecanol, the PEG-30 glyceryl stearate, the potassium cetyl phosphate and the pigments are mixed under hot conditions (approximately 95°C) with vigorous stirring.

The emulsion is then prepared by adding a part of the aqueous phase (water and acrylamide/sodium acryloyldimethyltaurate copolymer and preserving agents) heated to 85°C and then, after 10 minutes with vigorous stirring at 80°C, the rest of the aqueous phase, which has remained at ambient temperature, is added.

The mascara obtained is black, shiny and smooth. It has a threading nature under hot conditions, with threads of 20 mm on average. The threads obtained are fine, flexible and black.

**Example 3: Mascara**

1/ Preparation of a polymer of poly(isobornyl acrylate/isobutyl methacrylate/isobutyl acrylate)

100 g of isododecane are introduced into a 1-litre reactor and the temperature is then increased so as to go from ambient temperature (25°C) to 90°C in 1 hour.

120 g of isobornyl acrylate, 90 g of isobutyl methacrylate, 110 g of isododecane and 1.8 g of 2,5-bis(2-ethylhexanoylperoxy)-2,5-dimethylhexane (Trigonox® 141 from Akzo Nobel) are then added, at 90°C in 1 hour.
The mixture is maintained at 90°C for 1 h 30.
90 g of isobutyl acrylate, 90 g of isododecane and 1.2 g of 2,5-bis(2-ethylhexanoylperoxy)-2,5-dimethyl-hexane are then introduced into the above mixture, still at 90°C, and in 30 minutes.
The mixture is maintained at 90°C for 3 hours and the whole is then cooled.
A solution, containing 50% of active material, of polymer in isododecane is obtained.
A polymer comprising a first poly(isobornyl acrylate/isobutyl methacrylate) block or sequence having a Tg of 75°C, a second poly(isobutyl acrylate) block having a Tg of -20°C and an intermediate block, which is a random isobornyl acrylate/isobutyl methacrylate/isobutyl acrylate polymer, is obtained.
This polymer has a weight-average mass of 144 200 g/mol and a number-average mass of 49 300, i.e. a polydispersity index I of 2.93.
2/ The following mascara is prepared:

| | |
|---|---|
| Poly(isobornyl acrylate/isobutyl methacrylate/isobutyl acrylate) polymer | 58.54% |
| Pigments (iron oxide) | 2.44% |
| Isododecane | qs 100 |

The dmax of the composition is measured according to the protocol indicated above: this mascara has a dmax of approximately 12 mm.

**Example 4: Mascara**

| | |
|---|---|
| Styrene/butadiene copolymer (Pliolite S5E from Eliokem) | 30% |
| Copolymer of acrylamide/AMPS Na in isohexadecane with polysorbate 80 (Simulgel 600® from Seppic) | 2.4% |
| Polyethylene glycol stearate (30 EO) (Myrj 52 P from Uniquema) | 8.67% |
| Potassium cetyl phosphate at 79.5% in a mixture with phosphoric acid, cetyl alcohol and water (Arlatone MAP 160K from Givaudan) | 3.57% |
| Pigments (iron oxide) | 2.4% |
| Isododecane | 20% |
| Water | qs 100 |

This mascara has a threading nature dmax, measured according to the protocol indicated above, of approximately 24 mm.

**Example 5: mascara**

| | |
|---|---|
| Styrene/butadiene copolymer (Pliolite S5E from Eliokem) | 90% |
| Isododecane | 10% |

This mascara has a threading nature dmax, measured according to the protocol indicated above, of approximately 24 mm.

**Example 6: mascara**

| | |
|---|---|
| Sulphopolyester (Eastman AQ 38 S from Eastman Chemical) | 30% AM |
| Copolymer of acrylamide/AMPS Na in isohexadecane with polysorbate 80 (Simulgel 600® from Seppic) | 2.4% |
| Polyethylene glycol stearate (30 EO) (Myrj 52 P from Uniquema) | 8.67% |
| Potassium cetyl phosphate at 79.5% in a mixture with phosphoric acid, cetyl alcohol and water (Arlatone MAP 160K from Givaudan) | 3.57% |
| Butylene glycol | 20% |
| Pigments (iron oxide) | 2.4% |
| Water | qs 100 |

The dmax of the composition is measured according to the protocol indicated above: this mascara has a dmax of approximately 13 mm.

**Example 7: mascara**

| | |
|---|---|
| Sulphopolyester (Eastman AQ 38 S from Eastman Chemical) | 50% AM |
| Butylene glycol | 50% |

The dmax of the composition is measured according to the protocol indicated above: this mascara has a dmax of approximately 25 mm.

**Example 8: mascara**

A solid mascara comprising 100% of polyvinyl acetate homopolymer (Raviflex BL 1S from Vinavil) is prepared. This mascara has a threading nature dmax, measured according to the protocol indicated above, of approximately 29 mm.

The composition is placed on the heating part of a heating applicator, the composition softens, and then the mascara is applied to the eyelashes by drawing the deposit using the applicator.

**Example 9: mascara**

A mascara having the following composition was prepared according to the procedure of Example 1:

| | |
|---|---|
| Ethylene/octene copolymer (Affinity GA 1900 from Dow Plastics) | 33% |
| Octyldodecanol | 7.26% |
| Potassium cetyl phosphate at 79.5% in a mixture with phosphoric acid, cetyl alcohol and water (Arlatone MAP 160K from Givaudan) | 2.98% |
| PEG-30 glyceryl stearate | 7.26% |
| Copolymer of acrylamide/AMPS Na in isohexadecane with polysorbate 80 (Simulgel 600® from Seppic) | 0.8% AM |
| Preserving agents | qs |
| Black iron oxide | 2% |
| Water | qs 100% |

The dmax of the composition is measured according to the protocol indicated above: this mascara has a dmax of approximately 8 mm.

**Example 10: mascara**

A mascara having the following composition was prepared:

| | |
|---|---|
| Ethylene/vinyl acetate copolymer (Elvax 210W from Dupont) | 20% |
| Octyldodecanol | 8.67% |
| Potassium cetyl phosphate at 79.5% in a mixture with phosphoric acid, cetyl alcohol and water (Arlatone MAP 160K from Givaudan) | 3.57% |
| PEG-30 glyceryl stearate | 8.67% |
| Copolymer of acrylamide/AMPS Na in isohexadecane with polysorbate 80 (Simulgel 600® from Seppic) | 0.96% AM |
| Preserving agents | qs |
| Black iron oxide | 2.4% |
| Water | qs 100% |

This mascara is prepared according to the method indicated in Example 1.
The dmax of the composition is measured according to the protocol indicated above: this mascara has a dmax of approximately 10 mm.

**Example 11: mascara**

A mascara having the following composition was prepared:

| | |
|---|---|
| Ethylene/octene copolymer (Affinity GA 1900 from Dow Plastics) | 35% |
| Paraffin wax | 15% |
| Glycerol | 4.5% |
| Potassium cetyl phosphate at 79.5% in a mixture with phosphoric acid, cetyl alcohol and water (Arlatone MAP 160K from Givaudan) | 2.5% |
| Preserving agents | qs |
| Water | qs 100% |

The ethylene/octene copolymer, the paraffin, the glycerol and the potassium cetyl phosphate are mixed under hot conditions (approximately 95°C) with vigorous stirring.
The emulsion is then prepared by adding a part of the aqueous phase heated to 85°C and then, after 10 minutes with vigorous stirring at 80°C, the rest of the aqueous phase, which has remained at ambient temperature, is added. A white mascara is obtained.
The dmax of the composition is measured according to the protocol indicated above: this mascara has a dmax of approximately 7 mm.

**Example 12: mascara**

A mascara having the following composition was prepared:

| | |
|---|---|
| Ethylene/vinyl acetate copolymer (Elvax 205W from Dupont) | 41.47% |
| Hydrogenated styrene/methyl styrene/indene/- styrene copolymer (Regalite R1100 from Eastman) | 48.53% |
| Paraffin wax | 10% |

The ingredients are mixed at 140°C and the mixture is then allowed to cool to ambient temperature. A white-coloured solid mascara is obtained.
The dmax of the composition is measured according to the protocol indicated above: this mascara has a dmax of approximately 30 mm. After heating, this mascara forms fine, flexible and transparent threads.

**Example 13**

A mascara having the following composition was prepared:

| | |
|---|---|
| Coolbind 34-1300 from National Starch | 96% |
| Black iron oxide | 4% |

The ingredients are mixed at 100°C and the mixture is then allowed to cool to ambient temperature. A blackcoloured solid mascara is obtained.
The dmax of the composition is measured according to the protocol indicated above: this mascara has a dmax of approximately 35 mm.

## Example 14

A mascara having the following composition was prepared:

| | |
|---|---|
| Polyphenylsiloxane T resin | |
| (Dow Corning (R)Z-6018 intermediate) | 98% |
| Pigments | 2% |

The ingredients are mixed at 100°C and the mixture is then allowed to cool to ambient temperature. A coloured solid mascara is obtained.
The dmax of the composition is measured according to the protocol indicated above: this mascara has a dmax of approximately 25 mm.
After heating, this mascara forms coloured, rigid and thick threads.

## Example 15

The following mascara was prepared by melting the Coolbind and dispersing the pigments and the fibres, under hot conditions (95°C).

| | |
|---|---|
| Coolbind 34-1300 from National Starch | 95% |
| Pigment | 2% |
| Cellulose fibres 1.3 mm in length (Rayon Flock Rcise N0003 MO4 from Claremont Flock Corporation) | 3% |

The dmax of the composition is measured according to the protocol indicated above: this mascara has a dmax of approximately 28 mm.
After heating, this mascara forms coloured, rigid and thick threads.

## Example 16

The following mascara was prepared:

| | |
|---|---|
| Mixture of paraffin wax (30-50%)and ethylene/vinyle acetate copolymer(50 to 65%) | 95% |
| Iron oxide | 5% |

This mascara is prepared in a twin-screw mixer-extruder (of the prism type from Thermo Electron Corporation, England) comprising 6 independent barrels, each making it possible to introduce a new phase and to fix the temperature. They are numbered from 1 to 6 from the inlet to the outlet of the product. The flow rate is 2 kg/h (1000 rpm).
The 2 ingredients are introduced respectively into the first and second barrels, in which they are heated to 100°C then mixed under hot conditions.
The composition is then cooled in an ice cube box to obtain small solid blocks that are submitted to cryogenic grinding on a Quadro grinding device at -200 °C on a 0.5 mm sieve.
A composition under the form of a pulverulent mass is obtained.
This mascara has a threading nature under hot conditions, with threads of 35 mm on average.
The threads obtained are fine, rigid and black.

## Claims

1. Cosmetic process for making up or for the non-therapeutic care of keratin fibres, comprising the application to the keratin fibres of a cosmetic composition containing at least one compound or a mixture of compounds, which, when the composition is brought to a temperature of greater than or equal to 40°C, confers on said composition a threading

nature dmax of greater than or equal to 5 mm according to the measurement protocol of the description, said composition being, prior to, simultaneously with or subsequent to its application, brought to a temperature of greater than or equal to 40°C, the compound or the mixture of compounds which confers on said composition a threading nature dmax of greater than or equal to 5 mm is chosen from:

- copolymers of an alkene and of vinyl acetate, in particular copolymers of ethylene and of vinyl acetate;
- copolymers of ethylene and of octene,
- polyvinyl acetate homopolymers,
- silicone T resins, such as polyphenylsiloxanes,
- film-forming linear block polymer comprising at least isobornyl acrylate monomers, at least isobornyl methacrylate monomers and at least isobutyl acrylate monomers,,
- copolymers of butadiene and of styrene,
- copolymers obtained by condensation of diethylene glycol, cyclohexane dimethanol, isophthalic acid, sulphoisophthalic acid, and mixtures thereof.

**2.** Process according to Claim 1, **characterized in that** the composition has a dmax of greater than or equal to 7 mm, better still greater than or equal to 10 mm, and better still greater than or equal to 15 mm.

**3.** Process according to Claim 1 or 2, **characterized in that** the composition is brought to the temperature at which it has the threading nature dmax.

**4.** Process according to one of the preceding claims, **characterized in that** the composition is brought to a temperature of greater than or equal to 45°C, better still greater than or equal to 50°C, and even better still greater than or equal to 60°C.

**5.** Process according to one of the preceding claims, **characterized in that** the composition is brought to a temperature ranging up to 150°C, preferably up to 120°C, better still up to 100°C, even better still up to 95°C.

**6.** Process according to one of the preceding claims, **characterized in that** the compound or the mixture of compounds which confers on said composition a threading nature dmax of greater than or equal to 5 mm has a thermoplastic behaviour.

**7.** Process according to one of the preceding claims, **characterized in that** the compound or the mixture of compounds which confers on said composition a threading nature dmax of greater than or equal to 5 mm is chosen from copolymers of ethylene and of vinyl acetate.

**8.** Process according to one of the preceding claims, **characterized in that** the compound or the mixture of compounds which confers on said composition a threading nature dmax of greater than or equal to 5 mm is chosen from mixture of wax, and ethylene/vinyle acetate copolymer.

**9.** Process according to the preceding claim, **characterized in that** wax is paraffin wax.

**10.** Process according to claim 8 or 9, **characterized in that** the mixture comprises from 50 to 65% by weight of ethylene/vinyle acetate copolymer related to the total weight of the mixture.

**11.** Process according to any one of claims 8 to 10, **characterized in that** the mixture comprises from 30 to 50% by weight of wax related to the total weight of the mixture.

**12.** Process according to any one of claims 8 to 11, **characterized in that** the ethylene/vinyle acetate copolymer has a weight-average mass (Mw) ranging from 50000 to 80000, better, from 60 000 to 70 000, and even better from 63000 to 73000.

**13.** Process according to any one of claims 8 to 12, **characterized in that** the copolymer of ethylene and of vinyl acetate comprises more than 25% by weight of vinyl acetate relative to the total weight of the polymer.

**14.** Process according to one of the preceding claims, **characterized in that** the compound or the mixture of compounds which confers on said composition a threading nature dmax of greater than or equal to 5 mm is present at a solids content of greater than or equal to 5% by weight relative to the total weight of the composition.

**15.** Process according to one of the preceding claims, **characterized in that** the compound or the mixture of compounds which confers on said composition a threading nature dmax of greater than or equal to 5 mm is present at a solids content ranging from 5% to 100% by weight relative to the total weight of the composition, preferably ranging from 10% to 100% by weight, and better still from 12% to 100% by weight.

**16.** Process according to one of the preceding claims, **characterized in that** the composition comprises an aqueous phase.

**17.** Process according to the preceding claim, **characterized in that** the aqueous phase represents from 5% to 95% by weight, relative to the total weight of the composition.

**18.** Process according to one of the preceding claims, **characterized in that** the composition is a mascara.

**19.** Process according to one of the preceding claims, **characterized in that** the composition is in solid form.

**20.** Process according to one of the preceding claims, **characterized in that** the composition is brought to a temperature of greater than or equal to 40°C prior to, simultaneously with or subsequent to its application to the keratin fibres using an application device comprising heating means.

**21.** Process according to any one of the preceding claims, **characterized in that** the composition is applied to the upper end of the keratin fibres, in particular the eyelashes.

**22.** Packaging and application assembly for a composition for making up and/or for the care of keratin fibres, comprising:

i) a reservoir;
ii) a composition for making up and/or for the care of keratin fibres, placed inside the reservoir, said composition having a threading nature dmax of greater than or equal to 5 mm according to the measurement protocol of the description;
iii) a device for applying the makeup and/or care composition; and/or
iv) heating means for bringing said composition, simultaneously with or subsequent to its application, to a temperature of greater than or equal to 40°C, **characterized in that** the composition comprises a compound or the mixture of compounds which confers on said composition a threading nature dmax of greater than or equal to 5 mm is chosen from:

- copolymers of an alkene and of vinyl acetate, in particular copolymers of ethylene and of vinyl acetate;
- copolymers of ethylene and of octene,
- polyvinyl acetate homopolymers,
- silicone T resins, such as polyphenylsiloxanes,
- film-forming linear block polymer comprising at least isobornyl acrylate monomers, at least isobornyl methacrylate monomers and at least isobutyl acrylate monomers,,
- copolymers of butadiene and of styrene,
- copolymers obtained by condensation of diethylene glycol, cyclohexane dimethanol, isophthalic acid, sulphoisophthalic acid, and mixtures thereof.

**23.** Packaging and application assembly according to Claim 22, **characterized in that** the application device comprises heating means.

**Patentansprüche**

**1.** Kosmetisches Verfahren zum Schminken oder für die nichttherapeutische Pflege von Keratinfasern, umfassend das Auftragen einer kosmetischen Zusammensetzung auf die Keratinfasern, die mindestens eine Verbindung oder ein Gemisch von Verbindungen enthält, die bzw. das, wenn die Zusammensetzung auf eine Temperatur von mindestens 40 °C gebracht wird, der Zusammensetzung eine Fadenbildungseigenschaft dmax von mindestens 5 mm nach dem Messprotokoll der Beschreibung verleiht, wobei die Zusammensetzung vor, gleichzeitig mit oder nach ihrem Auftragen auf eine Temperatur von mindestens 40 °C gebracht wird, wobei die Verbindung oder das Gemisch von Verbindungen, die bzw. das der Zusammensetzung eine Fadenbildungseigenschaft dmax von mindestens 5 mm verleiht, ausgewählt ist aus:

- Copolymeren eines Alkens und von Vinylacetat, insbesondere Copolymeren von Ethylen und Vinylacetat;
- Copolymeren von Ethylen und Octen,
- Polyvinylacetathomopolymeren,
- Silikon-T-Harzen, wie beispielsweise Polyphenylsiloxanen,
- einem filmbildenden linearen Block-Copolymer, umfassend mindestens Isobornylacrylatmonomere, mindestens Isobornylmethacrylatmonomere und mindestens Isobutylacrylatmonomere,
- Copolymeren von Butadien und Styrol,
- Copolymeren, die durch Kondensation von Diethylenglycol, Cyclohexandimethanol, Isophthalsäure, Sulfoisophthalsäure erhalten werden, und Gemischen davon.

**2.** Verfahren nach Anspruch 1, **dadurch gekennzeichnet, dass** die Zusammensetzung eine dmax von mindestens 7 mm aufweist, noch besser von mindestens 10 mm und noch besser von mindestens 15 mm.

**3.** Verfahren nach Anspruch 1 oder 2, **dadurch gekennzeichnet, dass** die Zusammensetzung auf die Temperatur gebracht wird, bei der sie die Fadenbildungseigenschaft dmax aufweist.

**4.** Verfahren nach einem der vorhergehenden Ansprüche, **dadurch gekennzeichnet, dass** die Zusammensetzung auf eine Temperatur von mindestens 45 °C gebracht wird, noch besser auf mindestens 50 °C und noch besser auf mindestens 60 °C.

**5.** Verfahren nach einem der vorhergehenden Ansprüche, **dadurch gekennzeichnet, dass** die Zusammensetzung auf eine Temperatur im Bereich von bis zu 150 °C gebracht wird, vorzugsweise bis zu 120 °C, noch besser bis zu 100 °C, noch besser bis zu 95 °C.

**6.** Verfahren nach einem der vorhergehenden Ansprüche, **dadurch gekennzeichnet, dass** die Verbindung oder das Gemisch von Verbindungen, die bzw. das der Zusammensetzung eine Fadenbildungseigenschaft dmax von mindestens 5 mm verleiht, ein thermoplastisches Verhalten aufweist.

**7.** Verfahren nach einem der vorhergehenden Ansprüche, **dadurch gekennzeichnet, dass** die Verbindung oder das Gemisch von Verbindungen, die bzw. das der Zusammensetzung eine Fadenbildungseigenschaft dmax von mindestens 5 mm verleiht, aus Copolymeren von Ethylen und Vinylacetat ausgewählt wird.

**8.** Verfahren nach einem der vorhergehenden Ansprüche, **dadurch gekennzeichnet, dass** die Verbindung oder das Gemisch von Verbindungen, die bzw. das der Zusammensetzung eine Fadenbildungseigenschaft dmax von mindestens 5 mm verleiht, aus einem Gemisch aus Wachs und einem Ethylen/Vinylacetat-Copolymer ausgewählt wird.

**9.** Verfahren nach den vorhergehenden Ansprüchen, **dadurch gekennzeichnet, dass** es sich bei dem Wachs um Paraffinwachs handelt.

**10.** Verfahren nach Anspruch 8 oder 9, **dadurch gekennzeichnet, dass** das Gemisch relativ zu seinem Gesamtgewicht 50 bis 65 Gew.-% Ethylen/Vinylacetat-Copolymer umfasst.

**11.** Verfahren nach einem der Ansprüche 8 bis 10, **dadurch gekennzeichnet, dass** das Gemisch relativ zu seinem Gesamtgewicht 30 bis 50 Gew.-% Wachs umfasst.

**12.** Verfahren nach einem der Ansprüche 8 bis 11, **dadurch gekennzeichnet, dass** das Ethylen/Vinylacetat-Copolymer eine gewichtsgemittelte Masse (Mw) im Bereich von 50 000 bis 80 000 aufweist, besser von 60 000 bis 70 000 und noch besser von 63 000 bis 73 000.

**13.** Verfahren nach einem der Ansprüche 8 bis 12, **dadurch gekennzeichnet, dass** das Copolymer von Ethylen und Vinylacetat relativ zum Gesamtgewicht des Polymers mehr als 25 Gew.-% Vinylacetat umfasst.

**14.** Verfahren nach einem der vorhergehenden Ansprüche, **dadurch gekennzeichnet, dass** die Verbindung oder das Gemisch von Verbindungen, die bzw. das der Zusammensetzung eine Fadenbildungseigenschaft dmax von mindestens 5 mm verleiht, relativ zum Gesamtgewicht der Zusammensetzung als Feststoffgehalt von mindestens 5 Gew.-% vorhanden ist.

**15.** Verfahren nach einem der vorhergehenden Ansprüche, **dadurch gekennzeichnet, dass** die Verbindung oder das

Gemisch von Verbindungen, die bzw. das der Zusammensetzung eine Fadenbildungseigenschaft dmax von mindestens 5 mm verleiht, relativ zum Gesamtgewicht der Zusammensetzung als Feststoffgehalt im Bereich von 5 Gew.-% bis 100 Gew.-% vorhanden ist, vorzugsweise im Bereich von 10 Gew.-% bis 100 Gew.-% und noch besser von 12 Gew.-% bis 100 Gew.-%.

**16.** Verfahren nach einem der vorhergehenden Ansprüche, **dadurch gekennzeichnet, dass** die Zusammensetzung eine wässrige Phase umfasst.

**17.** Verfahren nach einem der vorhergehenden Ansprüche, **dadurch gekennzeichnet, dass** die wässrige Phase relativ zum Gesamtgewicht der Zusammensetzung 5 Gew.-% bis 95 Gew.-% repräsentiert.

**18.** Verfahren nach einem der vorhergehenden Ansprüche, **dadurch gekennzeichnet, dass** es sich bei der Zusammensetzung um Wimperntusche handelt.

**19.** Verfahren nach einem der vorhergehenden Ansprüche, **dadurch gekennzeichnet, dass** die Zusammensetzung in fester Form vorliegt.

**20.** Verfahren nach einem der vorhergehenden Ansprüche, **dadurch gekennzeichnet, dass** die Zusammensetzung vor, gleichzeitig mit oder nach ihrer Auftragung auf die Keratinfasern unter Verwendung einer Heizmittel aufweisenden Auftragungsvorrichtung auf eine Temperatur von mindestens 40 °C gebracht wird.

**21.** Verfahren nach einem der vorhergehenden Ansprüche, **dadurch gekennzeichnet, dass** die Zusammensetzung auf das obere Ende der Keratinfasern aufgetragen wird, insbesondere auf die Augenwimpern.

**22.** Verpackungs- und Auftragungsanordnung für eine Zusammensetzung zum Schminken und/oder für die Pflege von Keratinfasern, umfassend:

i) ein Reservoir;
ii) eine Zusammensetzung zum Schminken und/oder für die Pflege von Keratinfasern, die sich in dem Reservoir befindet, wobei die Zusammensetzung eine Fadenbildungseigenschaft dmax von mindestens 5 mm nach dem Messprotokoll der Beschreibung aufweist;
iii) eine Vorrichtung zum Auftragen der Makeup- und/oder Pflegezusammensetzung; und/oder
iv) Heizmittel, um die Zusammensetzung gleichzeitig mit oder nach ihrem Auftragen auf eine Temperatur von mindestens 40 °C zu bringen, **dadurch gekennzeichnet, dass** die Zusammensetzung eine Verbindung oder das Gemisch von Verbindungen umfasst, die bzw. das der Zusammensetzung eine Fadenbildungseigenschaft dmax von mindestens 5 mm verleiht, ausgewählt ist aus:

- Copolymeren eines Alkens und von Vinylacetat, insbesondere Copolymeren von Ethylen und Vinylacetat;
- Copolymeren von Ethylen und Octen,
- Polyvinylacetathomopolymeren,
- Silikon-T-Harzen, wie beispielsweise Polyphenylsiloxanen,
- einem filmbildenden linearen Block-Copolymer, umfassend mindestens Isobornylacrylatmonomere, mindestens Isobornylmethacrylatmonomere und mindestens Isobutylacrylatmonomere,
- Copolymeren von Butadien und Styrol,
- Copolymeren, die durch Kondensation von Diethylenglycol, Cyclohexandimethanol, Isophthalsäure, Sulfoisophthalsäure erhalten werden, und Gemischen davon.

**23.** Verpackungs- und Auftragungsanordnung nach Anspruch 22, **dadurch gekennzeichnet, dass** die Auftragungsvorrichtung Heizmittel aufweist.

## Revendications

**1.** Procédé cosmétique de maquillage ou de soin non thérapeutique de fibres kératiniques, comprenant l'application aux fibres kératiniques d'une composition cosmétique contenant au moins un composé ou un mélange de composés, qui, lorsque la composition est amenée à une température supérieure ou égale à 40°C, confère à ladite composition un caractère filant dmax supérieur ou égal à 5 mm selon le protocole de mesure de la description, ladite composition étant, avant, simultanément avec ou après son application, amenée à une température supérieure ou égale à 40°C,

le composé ou le mélange de composés qui confère à ladite composition un caractère filant dmax supérieur ou égal à 5 mm étant choisi parmi :

- des copolymères d'un alcène et d'acétate de vinyle, en particulier des copolymères d'éthylène et d'acétate de vinyle,
- des copolymères d'éthylène et d'octène,
- des homopolymères d'acétate de polyvinyle,
- des résines de silicone T, telles que les polyphénylsiloxanes,
- un polymère bloc linéaire filmogène comprenant au moins des monomères d'acrylate d'isobornyle, au moins des monomères de méthacrylate d'isobornyle, et au moins des monomères d'acrylate d'isobutyle,
- des copolymères de butadiène et de styrène,
- des copolymères obtenus par condensation de diéthylène glycol, de cyclohexane diméthanol, d'acide isophtalique, d'acide sulfoisophtalique, et des mélanges de ceux-ci.

**2.** Procédé selon la revendication 1, **caractérisé en ce que** la composition possède un dmax supérieur ou égal à 7 mm, préférablement supérieur ou égal à 10 mm, et préférablement supérieur ou égal à 15 mm.

**3.** Procédé selon la revendication 1 ou 2, **caractérisé en ce que** la composition est amenée à la température à laquelle elle possède le caractère filant dmax.

**4.** Procédé selon l'une des revendications précédentes, **caractérisé en ce que** la composition est amenée à une température supérieure ou égale à 45°C, préférablement supérieure ou égale à 50°C, et plus préférablement supérieure ou égale à 60°C.

**5.** Procédé selon l'une des revendications précédentes, **caractérisé en ce que** la composition est amenée à une température allant jusqu'à 150°C, préférablement jusqu'à 120°C, préférablement jusqu'à 100°C, plus préférablement jusqu'à 95°C.

**6.** Procédé selon l'une des revendications précédentes, **caractérisé en ce que** le composé ou le mélange de composés qui confère à ladite composition un caractère filant dmax supérieur ou égal à 5 mm, possède un comportement thermoplastique.

**7.** Procédé selon l'une des revendications précédentes, **caractérisé en ce que** le composé ou le mélange de composés qui confère à ladite composition un caractère filant dmax supérieur ou égal à 5 mm, est choisi parmi les copolymères d'éthylène et d'acétate de vinyle.

**8.** Procédé selon l'une des revendications précédentes, **caractérisé en ce que** le composé ou le mélange de composés qui confère à ladite composition un caractère filant dmax supérieur ou égal à 5 mm, est choisi parmi un mélange de cire, et de copolymère d'éthylène/acétate de vinyle.

**9.** Procédé selon la revendication précédente, **caractérisé en ce que** la cire est de la cire de paraffine.

**10.** Procédé selon la revendication 8 ou 9, **caractérisé en ce que** le mélange comprend de 50 à 65% en poids de copolymère d'éthylène/acétate de vinyle par rapport au poids total du mélange.

**11.** Procédé selon l'une quelconque des revendications 8 à 10, **caractérisé en ce que** le mélange comprend de 30 à 50% en poids de cire par rapport au poids total du mélange.

**12.** Procédé selon l'une quelconque des revendications 8 à 11, **caractérisé en ce que** le copolymère d'éthylène/acétate de vinyle possède une masse moyenne en poids (Mw) allant de 50 000 à 80 000, préférablement de 60 000 à 70 000, et plus préférablement de 63 000 à 73 000.

**13.** Procédé selon l'une quelconque des revendications 8 à 12, **caractérisé en ce que** le copolymère d'éthylène et d'acétate de vinyle comprend plus de 25% en poids d'acétate de vinyle par rapport au poids total du polymère.

**14.** Procédé selon l'une des revendications précédentes, **caractérisé en ce que** le composé ou le mélange de composés qui confère à ladite composition un caractère filant dmax supérieur ou égal à 5 mm, est présent selon une teneur en matières solides supérieure ou égale à 5% en poids par rapport au poids total de la composition.

15. Procédé selon l'une des revendications précédentes, **caractérisé en ce que** le composé ou le mélange de composés qui confère à ladite composition un caractère filant dmax supérieur ou égal à 5 mm, est présent selon une teneur en matières solides allant de 5% à 100% en poids par rapport au poids total de la composition, préférablement allant de 10% à 100% en poids, et plus préférablement de 12% à 100% en poids.

16. Procédé selon l'une des revendications précédentes, **caractérisé en ce que** la composition comprend une phase aqueuse.

17. Procédé selon la revendication précédente, **caractérisé en ce que** la phase aqueuse représente de 5% à 95% en poids par rapport au poids total de la composition.

18. Procédé selon l'une des revendications précédentes, **caractérisé en ce que** la composition est un mascara.

19. Procédé selon l'une des revendications précédentes, **caractérisé en ce que** la composition est sous forme solide.

20. Procédé selon l'une des revendications précédentes, **caractérisé en ce que** la composition est amenée à une température supérieure ou égale à 40°C avant, simultanément avec ou après son application aux fibres kératiniques à l'aide d'un dispositif d'application comprenant un moyen de chauffage.

21. Procédé selon l'une quelconque des revendications précédentes, **caractérisé en ce que** la composition est appliquée à l'extrémité supérieure des fibres kératiniques, en particulier aux cils.

22. Conditionnement et ensemble applicateur pour une composition de maquillage et/ou de soin de fibres kératiniques, comprenant :

    i) un réservoir ;
    ii) une composition de maquillage et/ou de soin de fibres kératiniques, placée à l'intérieur du réservoir, ladite composition ayant un caractère filant dmax supérieur ou égal à 5 mm selon le protocole de mesure de la description ;
    iii) un dispositif destiné à appliquer la composition de maquillage et/ou de soin ; et/ou
    iv) un moyen de chauffage destiné à amener ladite composition, simultanément avec ou après son application, à une température supérieure ou égale à 40°C, **caractérisé en ce que** la composition comprend un composé ou le mélange de composés qui confère à ladite composition un caractère filant dmax supérieur ou égal à 5 mm est choisi parmi :

    - des copolymères d'un alcène et d'acétate de vinyle, en particulier des copolymères d'éthylène et d'acétate de vinyle,
    - des copolymères d'éthylène et d'octène,
    - des homopolymères d'acétate de polyvinyle,
    - des résines de silicone T, telles que les polyphénylsiloxanes,
    - un polymère bloc linéaire filmogène comprenant au moins des monomères d'acrylate d'isobornyle, au moins des monomères de méthacrylate d'isobornyle, et au moins des monomères d'acrylate d'isobutyle,
    - des copolymères de butadiène et de styrène,
    - des copolymères obtenus par condensation de diéthylène glycol, de cyclohexane diméthanol, d'acide isophtalique, d'acide sulfoisophtalique, et des mélanges de ceux-ci.

23. Conditionnement et ensemble applicateur selon la revendication 22, **caractérisé en ce que** le dispositif d'application comprend un moyen de chauffage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1430868 A
- EP 1411069 A
- WO 04028488 A
- WO 04028493 A
- EP 847752 A
- FR 2232303 A
- US 5162410 A
- WO 2004073626 A
- WO 04055081 A
- US 6589331 B
- WO 06037900 A
- US 6009884 A
- US 5853010 A
- US 6220252 B


### Non-patent literature cited in the description


- Handbook of Pressure Sensitive Adhesives. 1989, 609-619
- Polymer Handbook. John Wiley, 1989
- *J. Soc. Cosm. Chem.,* 1954, vol. 5, 249-256
- Encyclopedia of Chemical Technology, Kirk-Othmer. Wiley, 1979, vol. 22, 333-432
- **E.M. FURST ; C. SUZUKI ; M. FERMIGIER ; A.P. GAST.** Permanently linked monodisperse paramagnetic chains. *Langmuir,* 1998, vol. 14, 7334-7336
- **M. FERMIGIER ; Y. GRASSELLI.** Suspensions de particules magnétiques. *Bulletin de la SFP [SFP bulletin,* July 1996
- **C. GOUBAULT ; P. JOP ; M. FERMIGIER ; J. BAUDRY ; E. BERTRAND ; J. BIBETTE.** Flexible magnetic filaments as micromechanical sensors. *Phys. Rev. Lett.,* 2003, vol. 91, 26